# FASCICULE DE BREVET EUROPEEN

(11) **EP 2 588 486 B1**
(45) Date de publication et mention de la délivrance du brevet: **31.05.2017**
(21) Numéro de dépôt: 11738049.3
(22) Date de dépôt: 24.06.2011
(51) Int. Cl.: C07F 17/02, C07D 319/06, B01J 27/185, B01J 31/18

(54) **LIGANDS SUPPORTES A HAUTE DENSITE LOCALE D'ATOMES COORDINANTS**
GETRÄGERTE LIGANDEN MIT HOHER LOKALER DICHTE AN KOORDINIERENDEN ATOMEN
SUPPORTED LIGANDS HAVING A HIGH LOCAL DENSITY OF COORDINATING ATOMS

(30) Priorité: 29.06.2010 FR 1002705
(43) Date de publication de la demande: 08.05.2013
(73) Titulaire: Centre National de la Recherche Scientifique, 75016 Paris (FR); Université de Bourgogne, 21078 Dijon Cedex (FR)
(72) Inventeur: HIERSO, Jean-Cyrille, F-21000 Dijon (FR); BEAUPERIN, Matthieu, 91120 Palaiseau (FR); MEUNIER, Philippe, F-21490 Bellefond (FR)
(74) Mandataire: Gevers & Orès
(86) Numéro de dépôt international: PCT/IB2011/052794
(87) Numéro de publication internationale: WO 2012/001601

(56) Documents cités:
- WO-A1-2006/117369
- WO-A2-2006/114438
- SU-A1- 709 625
- IVANOV V. V. , HIERSO J.-C., AMARDEIL R., MEUNIER P.: "General Route to Dissymetric Heteroannular-Functionalized Ferrocenyl 1,2-Diphosphines: Selective Synthesis and Characterization of a New Class of Tri- and Tetrasubstituted Ferrocenyl Compounds", ORGANOMETALLICS, vol. 25, 2006, pages 989-995, XP002619428,

## Description

L'invention concerne des composés, qui sont en particulier des ligands phosphiniques de complexes métalliques, ainsi que des complexes métalliques comprenant ces ligands et des catalyseurs supportés comprenant ces complexes métalliques ou ces composés.

Elle concerne également des procédés de synthèse de ces composés, complexes et catalyseurs supportés ainsi que des produits intermédiaires utilisés dans ces procédés de synthèse.

Elle concerne aussi l'utilisation des composés, selon l'invention, pour la fabrication d'un catalyseur supporté ou d'un complexe, selon l'invention pour la fabrication, également, d'un catalyseur supporté.

Enfin, elle concerne les procédés de couplage catalytique de composés nucléophiles et électrophiles basés sur l'utilisation de ces complexes et catalyseurs supportés.

La catalyse homogène à haute dilution est un procédé qui restreint considérablement la quantité de métal et de ligand utilisés dans les réactions catalytiques. C'est un procédé de synthèse plus propre qui a un fort potentiel dans l'industrie pharmaceutique, comme décrit par V. Farina (Adv. Synth. Catal. 2004, 346, 1553-1582).

WO 2006/117369 A1, WO 2006/114438 A2 et le document Ivanov et al., Organomet., 2006, 25,989-95 divulguent des dérivés de 1,2-diphosphineferrocène pouvant être utilisés pour la fabrication de catalyseurs utiles dans des procédés de couplage. Cependant, ces catalyseurs ne peuvent être utilisés qu'en catalyse homogène.

SU 709 625 A1 décrit des composés qui diffèrent des composés intermédiaires utilisés dans les procédés de synthèse de l'invention par l'absence du reste pentadiényle.

On connaît une famille de catalyseurs ayant prouvé leur efficacité en catalyse homogène à haute dilution (de 0,1 à 0,0001 % molaire de catalyseur par rapport au nombre de moles de réactif limitant).

Ces catalyseurs sont constitués d'un ligand qui est un composé polyphosphine ferrocénique à conformation contrôlée (voir M. Beaupérin et al. dans Eur. J. Inorg. Chem. 2007, 3767-3780) complexé avec un métal catalytique.

Les propriétés intéressantes de cette famille de catalyseurs sont dues à l'utilisation de ligands phosphines ferrocéniques à conformation contrôlée.

Ces ligands sont en particulier les suivants de formule (a) à (1) :

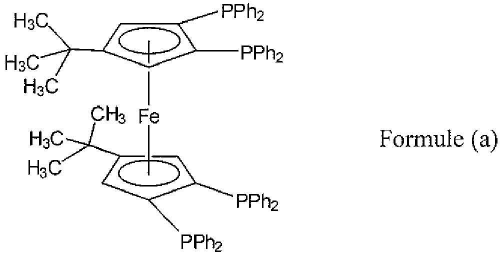

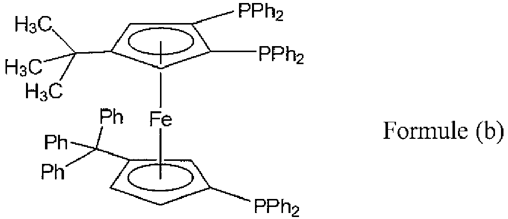

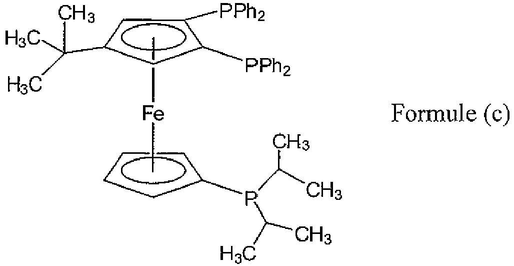

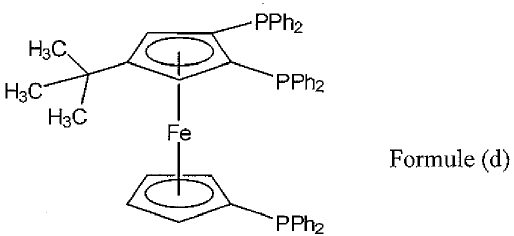

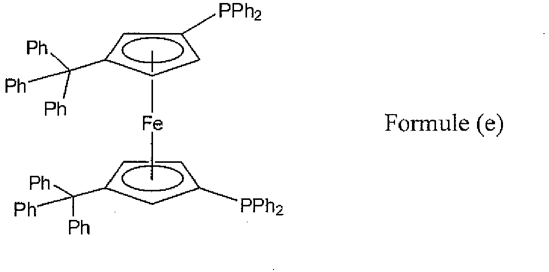

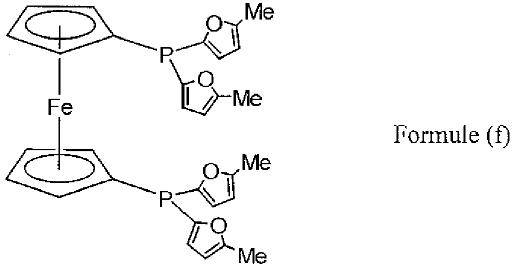

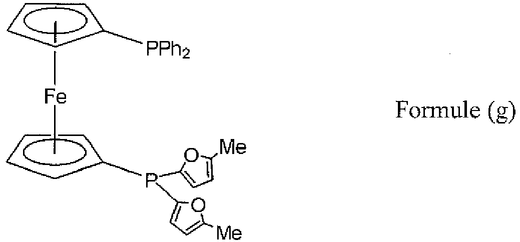

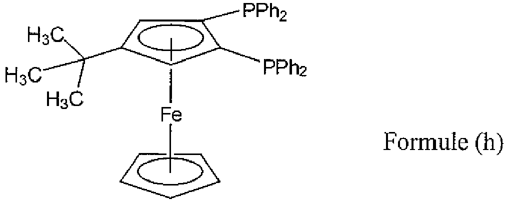

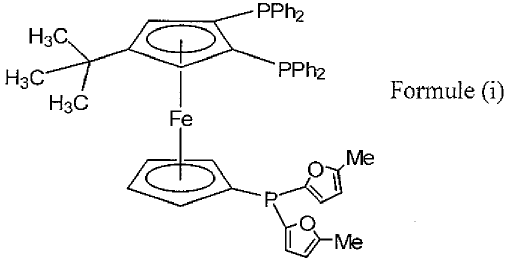

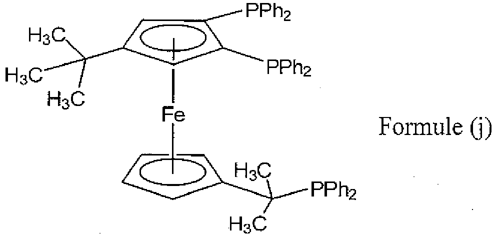

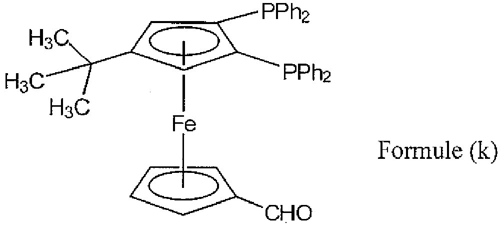

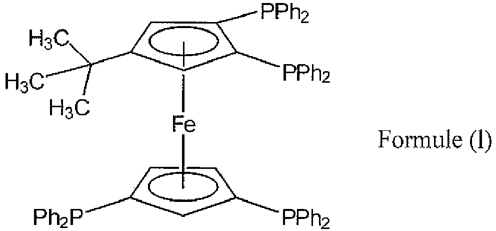

Les synthèses et les propriétés en catalyse à haute dilution de ces ligands est décrite en particulier dans : J.-C. Hierso et al. Organometallics 2003, 22, 4490-4499; J.-C. Hierso et al. Org. Lett. 2004, 6, 3473-3476; J.-C. Hierso et al. Tetrahedron 2005, 61, 9759-9766; A. Fihri et al. Adv. Synth. Catal. 2005, 347, 1198-1202, V.V. Ivanov et al. Organometallics 2006, 25, 989-995, R.V. Samliy et al. Organometallics 2009, 28, 3152-3160, J. Roger et al. ChemCatChem 2010, 2, 296-305.

Ces ligands, et en particulier le ligand de formule (a) qui est un ligand comportant quatre groupes phosphine, ont une conformation contrôlée, due au(x) groupement(s) tertiobutyle, (t-Bu), qui contrôle(nt) l'implantation des phosphores sur l'anneau cyclopentadiényle, et qui produit(sent) à la fois une excellente accessibilité et une grande proximité spatiale mutuelle des atomes de phosphore donneurs d'électrons qui sont susceptibles d'interagir en synergie avec un métal, et plus particulièrement le métal catalytique (comme démontré dans J.-C. Hierso et al. J. Am. Chem. Soc., 2004, 126, 11077-11087; J.-C. Hierso et al. Organometallics 2003, 22, 4490-4499 ; D. Evrard et al. Organometallics 2008, 27, 2643-2653).

Cependant, ces catalyseurs ne sont utilisables qu'en catalyse homogène.

Or, dans un but de chimie plus propre et dans le but de pouvoir réutiliser un catalyseur, en le recyclant, il est préférable d'utiliser une réaction de catalyse hétérogène.

Pour cela certains catalyseurs utilisés industriellement et en recherche académique sont dits être des catalyseurs « hétérogénéisés » (ou supportés), c'est-à-dire qu'ils sont immobilisés ou greffés sur un support solide (soluble ou insoluble), ou co-polymérisés pour fournir une résine coordinante (soluble ou insoluble).

Or, les ligands polyphosphines ferrocéniques décrits ci-dessus n'ont jamais, jusqu'à présent, pu être « hétérogénéisés », et cela est particulièrement vrai pour le composé de formule (a).

En effet, les sites disponibles sur les cycles pentadiènes de ce ligand polyphosphine ferrocénique ne sont pas accessibles sélectivement pour permettre leur fonctionnalisation directe dans le but d'hétérogéneiser le ligand ; hétérogénéisation à conduire par l'utilisation d'un support qui incorpore des groupes réactifs réagissant avec les fonctionnalisations, ou par l'immobilisation du ligand par polymérisation des groupes de fonctionnalisation entre eux, ou avec d'autres monomères.

D'autre part, il est essentiel de conserver la conformation de ces ligands, due aux groupes t-Bu, en particulier. Sur le ligand polyphosphine ferrocénique, ces groupes t-Bu ne sont pas eux-mêmes directement fonctionnalisables.

Or, les inventeurs ont maintenant réussi à créer des ligands polyphosphines ferrocéniques hétérogénéisables, c'est-à-dire comportant des groupes fonctionnels réactifs avec des groupes fonctionnels d'un support ou réactifs entre eux pour polymériser, ou encore réactifs avec d'autres monomères pour former un copolymère, tout en conservant la conformation de ces ligands, l'accessibilité des atomes de phosphore pour le métal réactif catalytique et la proximité des atomes de phosphore pour la stabilisation multiple de l'édifice catalytique.

Ainsi, l'invention est basée sur la synthèse de ligands phosphiniques et en particulier di-, tri- ou tétraphosphines ferrocéniques comportant à la place des groupements t-Bu des chaînes portant un groupe fonctionnel réactif lié à un espaceur lié à un groupe « structurant » mimant, en particulier l'effet du groupe tertiobutyle, qui est lui-même lié aux cycles pentadiène de la molécule de ferrocène.

Ces composés ont la formule (I) suivante : 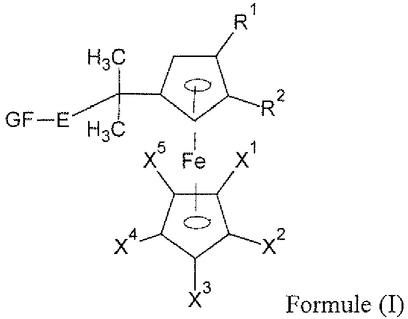 dans laquelle :
- E est une chaîne alkyle en C₁ à C₅ linéaire,
- GF est un groupe réactif choisi parmi les groupes vinyle et alkoxysilane, dont la chaîne alkoxy est en C₁ à C₅, linéaire ou ramifiée, saturée ou insaturée, contenant optionnellement au moins un hétéroatome, de préférence N et/ou O;
- R¹ est un groupe phosphine P(R⁴)₂,
- R² est H ou un groupe phosphine P(R⁶)₂,
- X¹, X², X³ et X⁴ sont identiques ou différents et sont choisis, indépendamment les uns des autres, parmi un atome d'hydrogène, un groupe (PR⁸)₂, un groupe -C(CH₃)₂-P(R³)₂, ou un groupe amino R⁷N(R⁵)₂,
- X₅ est soit un groupe GF-E-C(CH₃)₂-, dans lequel GF et E sont identiques à GF et E définis précédemment, soit H, soit un groupe phosphine P(R¹⁰)₂ ou un groupe amino R¹¹N(R⁹)₂
- R³, R⁴, R⁵, R⁶, R⁷, R⁸, R⁹, R¹⁰ et R¹¹ sont, indépendamment les uns des autres, choisis parmi :
   - un groupe alkyle en C₁ à C₃₀, de préférence en C₁ à C₁₀, plus préférablement en C₅ à C₇, linéaire ou ramifiée, saturée ou insaturée, contenant optionnellement au moins un hétéroatome, de préférence un atome de N et/ou O,
   - un groupe aryle, ou aliphatique cyclique ou acyclique, en C₃ à C₇, de préférence en C₁ à C₇ contenant optionnellement un hétéroatome, de préférence un atome de N et/ou O et optionnellement substitué, de préférence par un groupe méthyle, méthoxy, un atome d'halogène tel que F, Cl, Br ou I, ou un groupe CF₃.

De préférence, R³, R⁴, R⁵, R⁶, R⁸, R⁹ et R¹⁰ sont choisis parmi un groupe éthyle (Et), isopropyle (i-Pr), cyclohexyle (Cy), tertiobutyle (t-Bu), ou phényle (Ph), ou furyle (Fu), optionnellement substitués, de préférence par au moins un groupe méthyle, méthoxy, Cl, ou CF₃, et R⁷ et R¹¹ sont un groupe -CH₂-.

Le plus préférablement R⁴ et R⁶ sont un groupe phényle, R⁵ et R⁹ sont tous deux un groupe éthyle et R⁷ et R¹¹ sont tous deux un groupe -CH₂-.

Dans un premier mode de réalisation préféré, ces ligands, également appelés « composés » dans le présent texte, ont la formule (I-A) suivante : 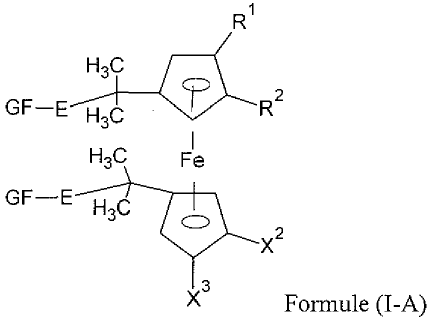 dans laquelle X⁵ est GF-E-C(CH₃)₂-.

Les composés préférés de formule (I-A) sont ceux dans lesquels X² = X³ = P(Ph₂) et X¹ = X⁴ = H et R¹ = R² = P(Ph)₂. Ces composés sont symétriques, de symétrie *C₂*.

Sont particulièrement préférés les composés de formules (1) à (8) suivantes :

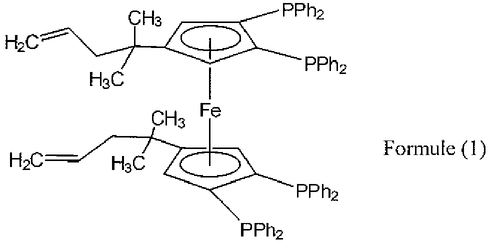

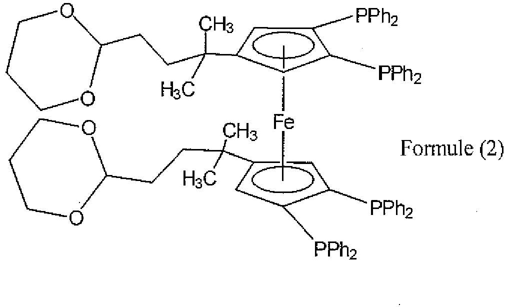

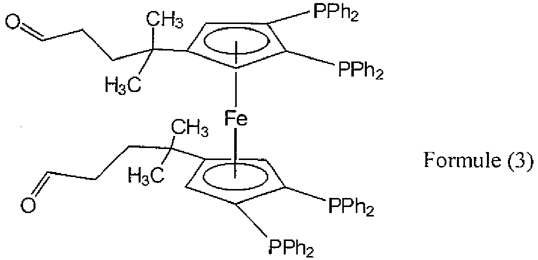

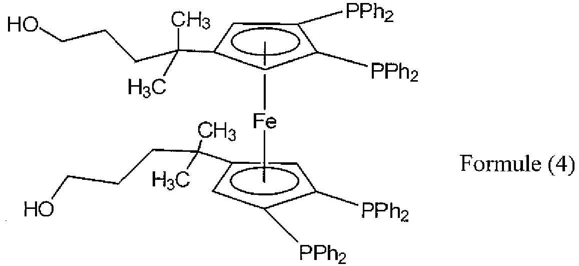

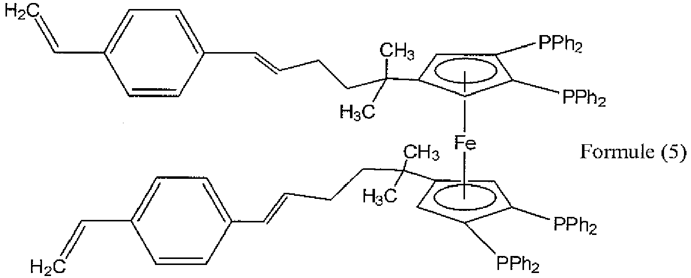

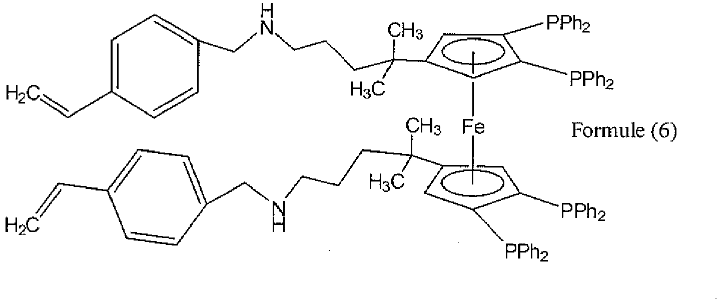

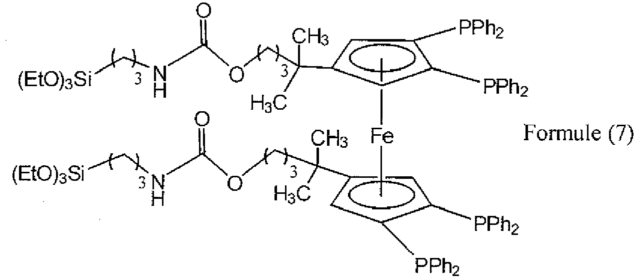

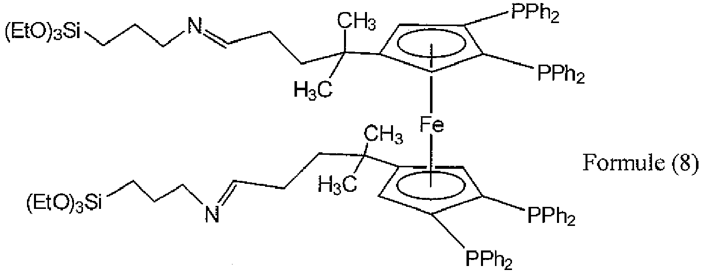

Dans un autre mode de réalisation préféré des composés de l'invention, les composés ont la formule (I) dans laquelle X⁵ est différent de GF-E-C(CH₃)₂-.

Ces composés sont dissymétriques.

Sont particulièrement préférés les composés de formules (9) à (18) suivantes : 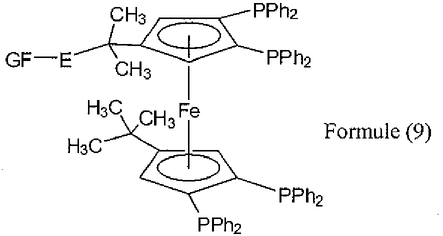 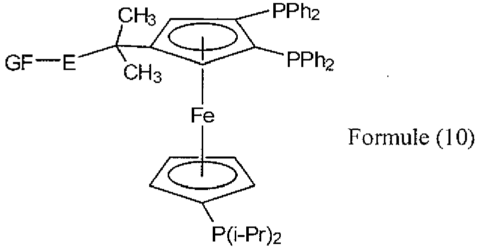 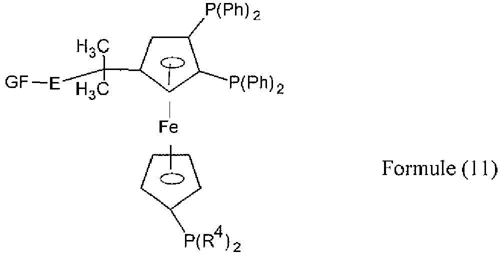 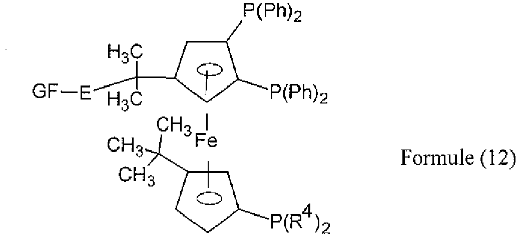 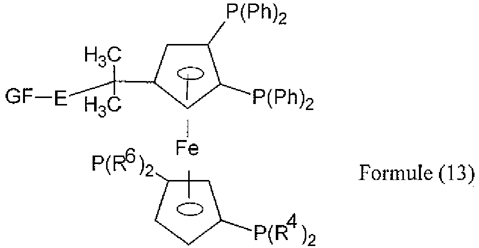 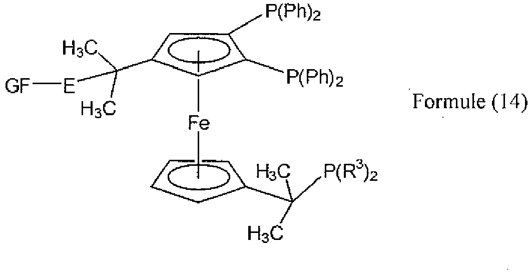 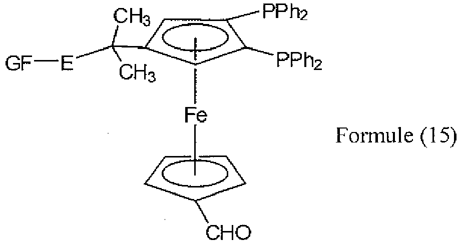 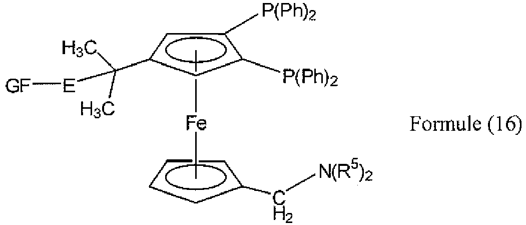 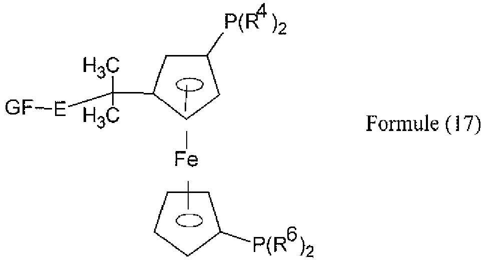 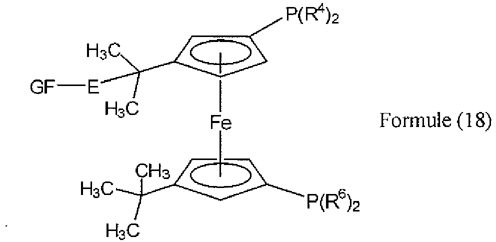 dans lesquelles :
- R⁴ et R⁶ désignent indépendamment les uns des autres un groupe isopropyle, cyclohexyle, tertiobutyle ou phényle, ou furyle, les groupes phényle ou furyle pouvant optionnellement être substitués, de préférence par au moins un groupe méthyle, méthoxy, un atome d'halogène, ou un groupe CF₃, et
- R⁵ désigne soit une chaîne alkyle en C₁ à C₃₀, de préférence en C₃ à C₁₀, plus préférablement en C₃ à C₅, linéaire ou ramifiée, saturée ou insaturée et pouvant contenir au moins un hétéroatome, de préférence N et/ou O, de préférence R⁵ désigne un groupe éthyle, soit un groupe aryle ou aliphatique, cyclique ou acyclique en C₅ à C₇, contenant optionnellement un hétéroatome et optionnellement substitué, de préférence par au moins un groupe méthyle, méthoxy, un atome d'halogène, ou un groupe CF₃.

De préférence, R⁴ et R⁶ sont tous deux un groupe phényle et R⁵ est un groupe éthyle.

Comme atome d'halogène on peut citer F, Cl, Br et I.

De préférence, l'atome d'halogène est Cl ou F.

Les composés-ligands de formules (1) à (9) sont des ligands tétraphosphines ferrocéniques.

Les composés-ligands de formules (10) à (14) sont des ligands triphosphines ferrocéniques.

Les composés-ligands de formules (15) à (18) sont des ligands diphosphines ferrocéniques.

Ce sont des ligands « hétérogénéisables » car le groupe GF permet soit leur greffage soit leur immobilisation au sein d'un support soluble récupérable (en fonction du solvant), ou d'un support insoluble.

La conformation des ligands de formules (a) à (1), connus, non « hétérogénéisables » correspondants, est conservée dans les ligands fonctionnalisés par les groupes GF-E-C(CH₃)₂- : c'est-à-dire les composés de formule (I), et plus particulièrement les composés de formules (1) à (9) de l'invention, comme cela a été vérifié par RMN multinoyaux ¹H, ¹³C, ³¹P et leur étude par diffraction des rayons X, à différentes étapes de leur synthèse.

Les ligands hétérogénéisables de l'invention sont ensuite immobilisés soit par greffage sur un support solide à fonctions réactives, soit par polymérisation de leurs groupes fonctionnels, entre eux, ou avec d'autres monomères.

En tant que support solide, on peut utiliser les supports solides connus de l'homme du métier tels que les résines, les polymères, la silice, l'alumine, les dendrimères, les nanoparticules métalliques ou de latex, les matériaux mésoporeux, les argiles, etc.

La réaction d'immobilisation sur le support se fait par réaction du groupe GF des composés de formule (I) et plus particulièrement des composés de formules (1) à (18), avec des groupes réactifs présents ou introduits sur le support.

Ainsi :
- lorsque le groupe GF est C = O, il réagira avec les groupes réactifs NH₂ du support, et inversement lorsque le groupe GF est un groupe NH₂, il réagira avec les groupes C = O du support,
- lorsque le groupe GF est OH, il réagira avec les groupes réactifs halogène (I, Br, Cl), siloxane (SiOR₃), mésylate (Ms), triflate (OTf) ou tosylate (OTs) du support, et inversement
- lorsque le groupe GF est -CH=CH₂, il réagira avec les groupes réactifs halogène (I, Br, Cl), siloxane (SiOR) du support, et inversement.

Les ligands de l'invention peuvent également être immobilisés par polymérisation de leurs groupes fonctionnels. Cela est le cas, en particulier lorsque ces groupes fonctionnels GF sont des groupes styrènyle ou siloxane comme par exemple, les groupes GF-1, GF-2 et GF-3 :

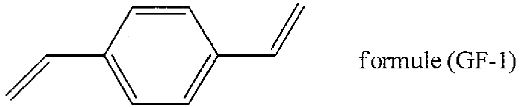

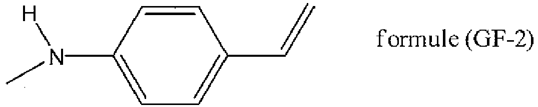

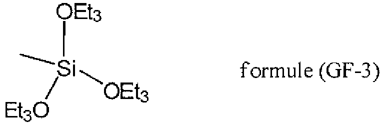

Ils peuvent être immobilisés par co-polymérisation de leurs groupes fonctionnels GF avec d'autres monomères organiques ou inorganiques, de même type oléfinique ou alicoxysilane.

Par exemple, lorsque le groupe GF a la formule GF-1 ou GF-2, il peut être co-polymérisé avec les monomères de formule (I-1) suivante : 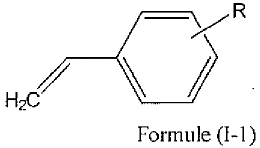 dans lesquels R est :
- Hou
- au moins un groupe alkyle à chaîne saturée ou insaturée en C₁ à C₃₀, linéaire ou ramifiée, préférentiellement en C₁ à C₅, ou
- au moins un groupe méthoxy, ou
- au moins un halogène tel que F, Cl, Br, I et préférentiellement F et Cl.

Lorsque le groupe GF est le groupe GF-3, il peut être co-polymérisé avec un monomère alkoxysilane de formule (SiOR'₃) dans lequel R' est un groupe alkyle à chaîne saturée ou insaturée en C₁ à C₃₀, linéaire ou ramifiée, préférentiellement en C₁ à C₅.

Les ligands de l'invention forment des complexes avec un métal catalytique comme décrit pour les ligands non-hétérogénéisables correspondants, dans J.-C. Hierso et al. Chem. Soc. Rev. 2007, 36, 1754-1769. La synthèse des complexes peut se faire, soit avant l'immobilisation du ligand sur le support, soit après, selon le schéma suivant.

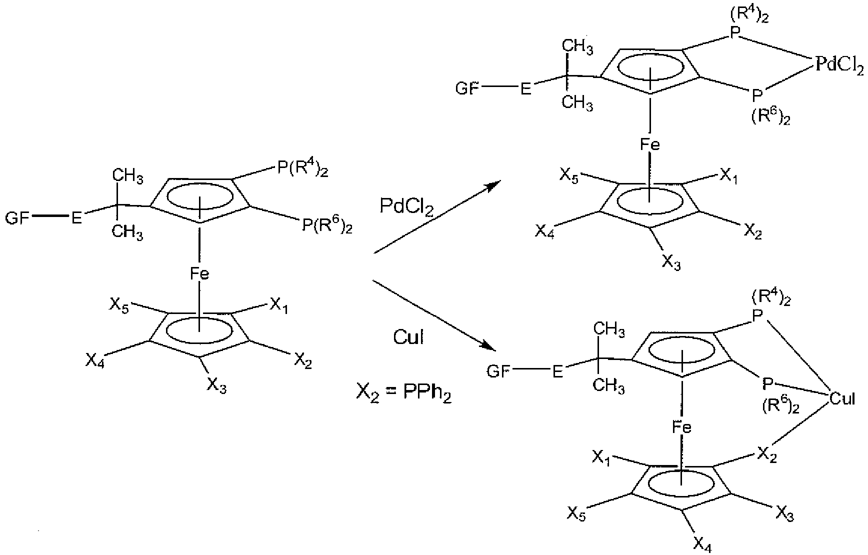

Dès lors, l'invention propose des complexes comprenant un composé de formule (I), de préférence un composé-ligand de formules (1) à (18) ci-dessus, complexé avec un métal choisi parmi les métaux de transition d, et en particulier le palladium, le cuivre, le nickel, le platine, le rhodium, l'iridium, le fer, le zirconium, le titane et le ruthénium ainsi que des complexes supportés comprenant un complexe selon l'invention immobilisé, tel que défini ci-avant, par greffage des groupes fonctionnels GF du composé de formule (I) avec les groupes réactifs d'un support, préférentiellement parmi des composés-ligands de formules (1) à (18), avec les groupes réactifs d'un support ou par polymérisation des groupes fonctionnels GF des composés de formule (I), préférentiellement parmi des composés-ligands de formules (1) à (18), entre eux ou avec d'autres monomères.

Un procédé de synthèse d'un composé de formule (I-A), en particulier ayant l'une des formules (1) à (8) est également un objet de l'invention.

Ce procédé comprend les étapes suivantes :
a) préparation d'un sel de lithium cyclopentadiénylique portant les groupements E-GF désirés, ou portant une fonction réactive précurseur conduisant facilement à ces groupements E-GF désirés une fois le ligand formé,
b) deux réactions successives de phosphination/lithiation du composé obtenu à l'étape a), et
c) formation du composé ferrocène désiré par réaction du composé obtenu à l'étape b) avec un sel de fer.

L'invention propose ainsi un procédé de synthèse du composé de formule (1), comprenant les étapes suivantes :
a) introduction du groupement fonctionnel GF : -CH=CH₂ et de l'espaceur E : -CH₂- par réaction du 6,6-diméthylfulvène avec l'organomagnésien CH₂=CH-CH₂-MgCl pour obtenir le 1-[2-(2-méthyl)pent-4-ényl]cyclopentadiène,
b) lithiation du produit obtenu à l'étape a) avec *n*-BuLi pour obtenir le 1-[2-[2-méthyl]pent-4-ényl]cyclopentadiényllithium,
c) deux phosphinations successives du produit obtenu à l'étape b) avec ClP(Ph)₂, entre lesquelles on réalise une étape de lithiation avec *n*-BuLi,
d) traitement du composé obtenu à l'étape c) avec n-BuLi pour obtenir le 1,2-bis(diphénylphosphino)-4-[2-(2-méthyl)pent-4-ényl]cyclopentadiényllithium, et
e) synthèse du composé de formule (1) par mise en réaction de FeCl₂ avec le composé obtenu à l'étape d).

Un autre objet de l'invention est un procédé de synthèse du composé de formule (2) comprenant les étapes suivantes :
a) réaction d'un organomagnésien de formule suivante : 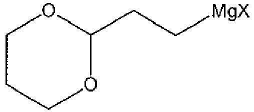 dans laquelle X est Br ou I
   avec CH₃COCl, pour obtenir la 4-(1,3-dioxane-2-yl)butane-2-one de formule A suivante : 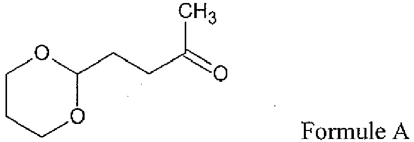
b) réaction du composé de formule A obtenu à l'étape a), avec de la pyrrolidine et du cyclopentadiényllithium (CpLi) pour obtenir le 2-[3-(cyclopenta-2,4-diénylidène)butyl]-1,3-dioxane de formule B suivante : 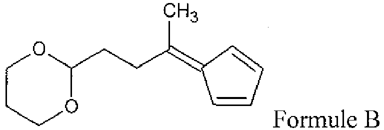
c) lithiation du composé de formule B avec du méthyllithium (MeLi) pour obtenir le composé de formule C suivante : 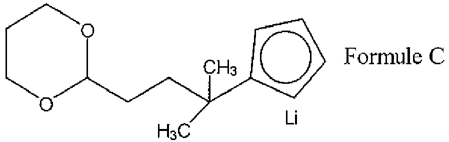
d) deux phosphinations successives du composé de formule C avec ClP(Ph)₂, avec passage intermédiaire au sel de lithium correspondant à la monophosphination par action de *n*-BuLi, suivies par un traitement avec *n*-BuLi pour obtenir le composé de formule D suivante : 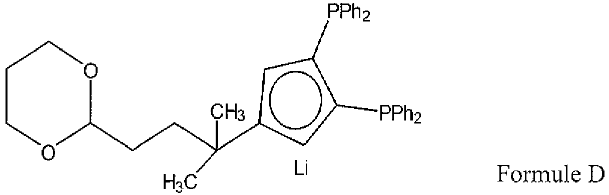
e) formation du composé de formule (2) suivante : 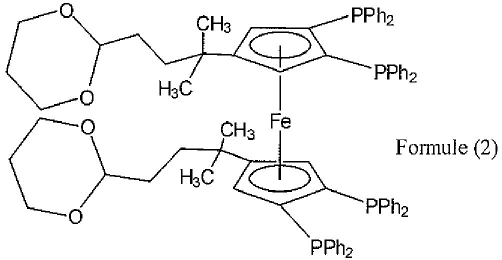 par réaction du composé de formule D avec un sel de fer.

Il est à noter qu'à l'étape d) entre les deux étapes de phosphination, on met en oeuvre une étape de lithiation du produit obtenu après la première étape de phosphination, avec *n*-BuLi.

L'invention propose également un procédé de synthèse du composé de formule (3) qui comprend la déprotection du composé de formule (2) dans des conditions acides.

A partir du composé de formule (3), l'invention propose un procédé de synthèse du composé de formule (4) qui comprend la réduction de ce composé de formule (3).

Le composé de formule (5) peut être fabriqué à partir du composé de formule (3) qui est mis à réagir avec un composé de formule (1-4) suivante :

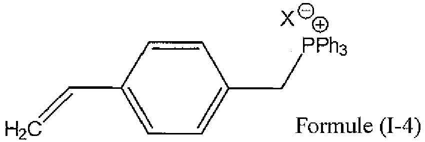

Mais le composé de formule (5) peut également être préparé par réaction du composé de formule (3) avec le composé de formule (I-5) suivante :

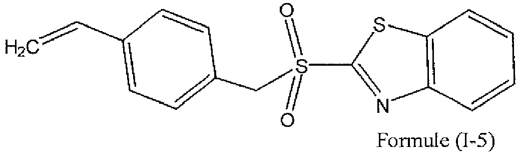

Quant au composé de formule (6), l'invention propose de le synthétiser par réaction du composé de formule (3) avec un composé *p*-vinylbenzylamine de formule (I-2) suivante :

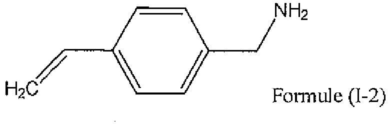

Le composé de formule (7) peut avantageusement être synthétisé, selon l'invention, par la réaction du composé de formule (4) avec un composé alkoxysilane de formule (1-6) suivante :

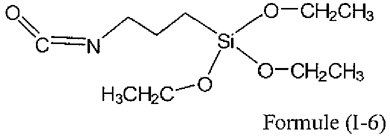

Le composé de formule (8) peut être synthétisé par la réaction du composé de formule (3) avec un composé alkoxysilane de formule (I-7) suivante :

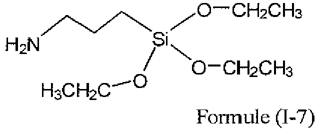

Quant aux composés de formules (9) à (16), ils peuvent être fabriqués par un procédé de synthèse qui comprend les étapes suivantes :
a) synthèse du composé de formule D suivante : 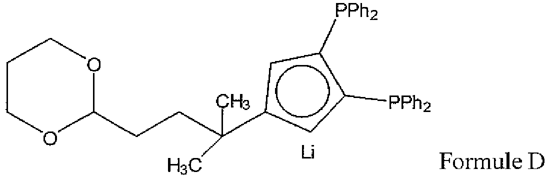
b) synthèse d'un sel d'un métal M, de préférence lithium ou sodium, de cyclopentadiényle ayant la formule G suivante : 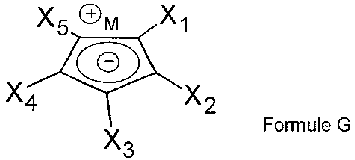 dans laquelle X¹, X², X³, X⁴ et X⁵ sont tels que définis précédemment pour les composés de formule (I) dissymétriques,
c) réaction du composé obtenu à l'étape b) avec un sel de fer et le composé D.

Plus précisément, le composé de formule (9) est synthétisé, selon l'invention, par un procédé qui comprend les étapes de réaction du procédé général de synthèse des composés de formules (9) à (16) décrits ci-dessus et en utilisant un composé de formule G dans lequel X¹ = X⁴ = H, X² = X³ = PPh₂, X⁵ = t-Bu et M = Li.

Le composé de formule (10) peut être fabriqué selon le procédé général de synthèse des composés de formules (9) à (16) décrit ci-dessus en utilisant un composé E dans lequel X¹ = X² = X⁴ = X⁵ = H , X³ = P(i-Pr)₂ et M = Li.

Le composé de formule (11) peut être fabriqué, selon l'invention, par un procédé qui comprend des étapes de réaction du procédé de synthèse général des composés de formules (9) à (16) en utilisant, un composé G dans lequel M = Li, X¹ = X² = X⁴ = X⁵ = H, et X³ = P(R⁴)₂ dans lequel R⁴ est choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O. De préférence R⁴ est un groupe cyclohexyle, tertiobutyle, ou phényle, ou furyle optionnellement substitués, de préférence par au moins un groupe méthyle, méthoxy, un atome d'halogène tel que F, Cl, Br, I, de préférence F ou Cl, ou un groupe CF₃.

Le composé de formule (12) peut être synthétisé, selon l'invention, par le procédé de synthèse général des composés de formules (9) à (16) précédemment décrit en utilisant un composé G dans lequel M = Li, X¹ = X² = X⁴ = H, X³ = P(R⁴)₂, X⁵ = t-Bu avec un groupe R⁴ choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O. De préférence R⁴ est un groupe isopropyle, cyclohexyle, tertiobutyle, ou phényle, ou furyle optionnellement substitués, de préférence par au moins un groupe méthyle, méthoxy, ou un atome d'halogène tel que F, Cl, Br, I, de préférence F ou Cl, ou un groupe CF₃.

Le composé de formule (13) peut être synthétisé, selon l'invention, par un procédé qui comprend les étapes de réaction du procédé de synthèse des composés de formules (9) à (16) décrits ci-dessus, en utilisant un composé G dans lequel M = Li, X¹ = X² = X⁴ = H, X³ = P(R⁴)₂, X⁵ = P(R⁶)₂, dans lesquels R⁴ et R⁶ sont, indépendamment l'un de l'autre, choisis parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O. De préférence R⁴ et R⁶ sont indépendamment l'un de l'autre un groupe isopropyle, cyclohexyle, tertiobutyle, ou phényle, ou furyle optionnellement substitués, de préférence par au moins un groupe méthyle, ou méthoxy ou un atome d'halogène tel que F, Cl, Br, I, de préférence F ou Cl, ou un groupe CF₃.

Le composé de formule (14) peut être fabriqué, par un procédé selon l'invention qui comprend les réactions du procédé de synthèse général des composés de formules (9) à (16) décrit ci-dessus, en utilisant un composé G dans lequel X¹ = X² = X⁴ = X⁵ = H, X³ = -C(CH₃)₂-P(R³)₂ et M = Li ; R³ est choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O. De préférence R³ est un groupe isopropyle, cyclohexyle, tertiobutyle, ou phényle, ou furyle optionnellement substitués, de préférence par au moins un groupe méthyle, méthoxy, halogène (F, Cl, Br, I) de préférence F ou Cl, ou CF₃. Plus préférablement, R³ est un groupe phényle substitué ou non.

Le composé de formule (15) peut être fabriqué, selon l'invention, par le procédé de synthèse décrit précédemment pour les composés de formules (9) à (16) en utilisant un composé G dans lequel X¹ = X² = X⁴ = X⁵ = H, X³ = CHO et M = Na.

Le composé de formule (16) peut être fabriqué, selon l'invention par la réaction du composé de formule (15) avec une amine secondaire en présence de NaBH(OAc)₃, l'amine secondaire ayant la formule HN(R⁵)₂ dans laquelle R⁵ est un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O. De préférence R⁵ est un groupe éthyle, isopropyle, cyclohexyle, tertiobutyle, ou phényle, ou furyle optionnellement substitués, de préférence par au moins un groupe méthyle, ou méthoxy ou un atome d'halogène tel que F, Cl, Br, I, de préférence F ou Cl, ou un groupe CF₃. R⁵ est, plus préférablement, un groupe éthyle.

Les composés de formules (17) et (18) peuvent être fabriqués par un procédé de synthèse qui comprend les étapes suivantes :
a) synthèse du composé de formule F suivante : 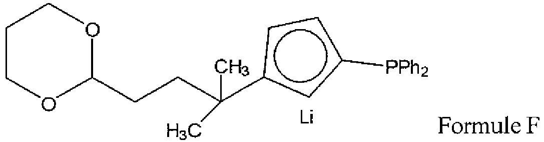
b) synthèse d'un sel d'un métal M, de préférence lithium ou sodium, de cyclopentadiényle ayant la formule G suivante : 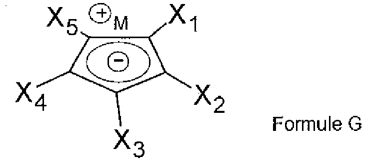 dans laquelle X¹, X², X³, X⁴ et X⁵ sont tels que définis précédemment dans les composées de formule (9) à (14), pour les composés de formules (17) ou (18) dissymétriques,
c) réaction du composé obtenu à l'étape b) avec un sel de fer et le composé F.

Lors de la synthèse des composés selon l'invention, les composés intermédiaires A et B, C, D et F ont été synthétisés.

A la connaissance des inventeurs, ces composés n'étaient pas connus jusqu'à ce jour.

Ils sont donc également un objet de l'invention.

Ainsi, l'invention concerne :
- un composé de formule A suivante : 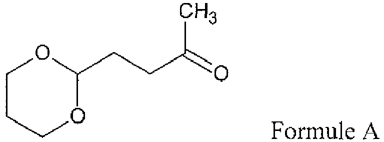
- un composé de formule B suivante : 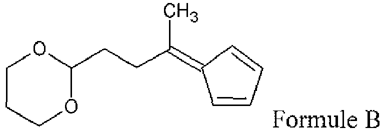
- un composé de formule C suivante : 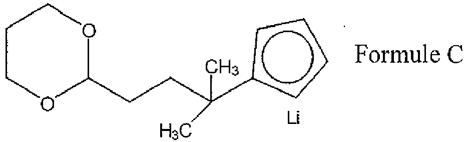
- un composé de formule D suivante : 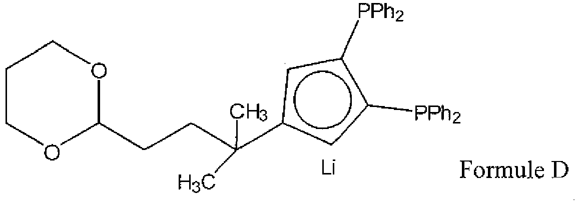
- un composé de formule F suivante :

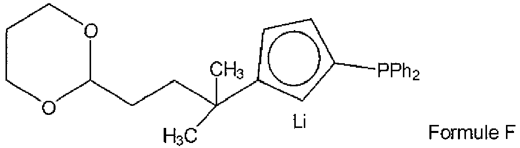

Les composés-ligands supportés de l'invention, et en particulier les composés (1) à (18) sont ensuite :
- soit complexés avec un métal catalytique, et ensuite immobilisés sur un support par greffage des groupes réactifs du composé ligand concerné avec les groupes réactifs du support,
- soit d'abord immobilisés sur le support et ensuite complexés avec le métal catalytique.

La complexation du composé-ligand selon l'invention, ou du composé-ligand supporté selon l'invention, se fait par mise en solution ou en suspension dans un milieu solvatant classique (préférentiellement CH₂Cl₂, CHCl₃, toluène, acétonitrile, diméthylformamide, dioxane, méthanol, éthanol, eau), sous forte agitation, d'un précurseur des complexes des métaux de transition (comme mentionné précédemment, par exemple sel de palladium PdCl₂ ou sel de cuivre CuI) et réaction avec le composé-ligand ou le composé-ligand supporté dans ce milieu, à température ambiante (entre 15 et 25 °C) si le milieu est homogène, ou a température entre 50 et 80 °C si le milieu est hétérogène pour faciliter la complexation.

Ainsi, un composé-ligand selon l'invention est particulièrement adapté pour la fabrication d'un catalyseur supporté, par complexation avec un métal catalytique.

Les complexes ou catalyseurs supportés selon l'invention, ainsi obtenus, se sont révélés particulièrement adaptés dans les procédés de couplage catalytique de composés nucléophiles ou électrophiles, tels qu'un couplage carbone-carbone, ou carbone-azote, ou carbone-oxygène.

Afin de mieux faire comprendre l'invention, on va maintenant en décrire, à titre purement illustratifs et non limitatifs, des exemples de mise en oeuvre.

### Exemple 1 : Synthèse du composé de formule (1)

Le composé de formule (1) est un ligand tétraphosphine ferrocénique fonctionnalisé par un groupement vinyle.

Dans ce composé, l'espaceur est un chaînon -CH₂- et le groupement fonctionnel est le groupement vinyle -CH=CH₂.

Ce composé a été synthétisé de la manière suivante :

### a) Introduction de la double liaison C=C

La première étape consiste à introduire la double liaison désirée sur le 6,6-diméthylfulvène de formule suivante :

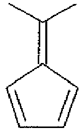

La réaction entre ce fulvène et le bromure d'allyle magnésien a déjà été décrite par Vos et Jutzi dans Synthesis 2000, 3, 357 et permet d'obtenir facilement le fragment cyclopentadiène avec 60% de rendement après purification. Cette méthode présente l'avantage d'utiliser des réactifs simples : le 6,6-diméthylfulvène est synthétisé en grandes quantités au laboratoire, et le bromure d'allyle magnésium est disponible commercialement (2M dans le THF).

Le diène a été obtenu après distillation (67 °C à 25 mm Hg) avec 60% de rendement. Sa lithiation dans l'hexane par du *n*-BuLi (1,6 M dans l'hexane) à -80 °C conduit au 1-[2-(2-méthyl)pent-4-ényl]cyclopentadiényle lithium sous forme d'une poudre blanche avec 98 % de rendement. Celui-ci a été caractérisé par RMN ¹H par l'apparition d'un système de spin de type vinylique : deux doublets dédoublés à 4,91 et 5,03 ppm et un multiplet à 5,92 ppm.

### b) Phosphinations du 1-[2-(2-méthyl)pent-4-ényl]cyclopentadiényllithium

Le 1-[2-(2-méthyl)pent-4-ényl]cyclopentadiényle a ensuite été engagé dans des réactions de phosphination. Celles-ci ont été menées de manière classique. La présence du pseudo *tert*-butyle (*gem*-diméthyle) permet d'orienter la disubstitution des phosphines en 1,2 plutôt qu'en 1,3 qui est préférée lorsqu'il n'y a pas de substitution préalable des positions du cycle.

La première phosphination conduit au 1-diphénylphosphino-3-[2-(2-méthyl)pent-4-ényl]cyclopentadiényllithium sous forme d'un solide blanc cassé, avec 90% de rendement, caractérisé en RMN ³¹P par un singulet à -21,1 ppm.

La deuxième phosphination se déroule dans les mêmes conditions et permet d'obtenir le 1,2-bis(diphénylphosphino)-4-[2-(2-méthyl)pent-4-ényl]cyclopentadiényllithium sous forme d'un solide blanc cassé, avec 80% de rendement global, caractérisé en RMN ³¹P par un singulet à -22,5 ppm.

### c) Synthèse du 1, 1', 2, 2'-tetrakis(diphénylphosphino)-4,4'-bis[2-(2-méthylpent-4-ényl)]ferrocène

Le composé obtenu à l'étape b) a ensuite été opposé à 0,5 équivalent de FeCl₂ pour donner après 24 h au reflux du toluène et purification par chromatographie sur gel de silice (toluène/hexane 1 :1 puis 2 :1) un solide rouge avec 20 % de rendement.

Le FeCl₂ utilisé est sous forme de billes. Afin d'augmenter l'efficacité de la réaction, les billes ont été préalablement écrasées par agitation magnétique, dans le solvant de réaction quelques heures avant l'addition du lithien de manière à obtenir une poudre fine.

Le dérivé ferrocénique de formule (1) a été caractérisé par RMN ¹H, ³¹P, ¹³C, et DRX. Les spectres RMN ¹H et ¹³C sont en accord avec la structure attendue. Le spectre RMN ³¹P montre deux multiplets centrés sur -30,6 ppm et 34,5 ppm typiques du système de spin AA'BB' attendu.

L'obtention de ce spectre indique que la conformation du ligand a bien été conservée. Des cristaux analysables en DRX ont été obtenus par diffusion dans de l'hexane d'une solution du ligand dans du toluène. La structure obtenue permet de confirmer que les anneaux cyclopentadiényle se positionnent dans une conformation identique à celle trouvée pour le ligand non fonctionnalisé correspondant.

### Exemple 2 : Synthèse des composés de formule (2) et (3)

Le composé de formule (2) est un ligand tétraphosphine ferrocénique fonctionnalisé par un groupe fonctionnel cétonique. Deux voies utilisant les composés organométalliques ont été utilisées pour synthétiser ce ligand.

La première voie utilise un composé organozincique et la seconde voie utilise un composé organomagnésien. Dans la première voie, la première étape consiste en la formation d'un organozincique puis son couplage de type Negishi avec le chlorure d'acétyle.

La synthèse d'organozincique par l'insertion directe de zinc avec des bromures et des iodures grâce à l'addition de chlorure de lithium a été décrite par Knochel et al. dans Angew. Chem. Int. Ed. 2006, 45, 6040.

Cette méthode a été appliquée selon le schéma général suivant :

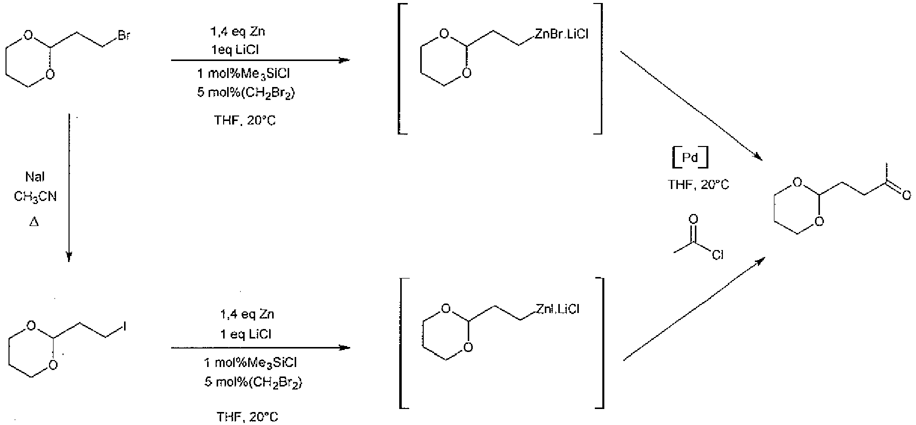

On obtient ainsi la 4-(1,3-dioxane-2-yl)butane-2-one de formule A suivante :

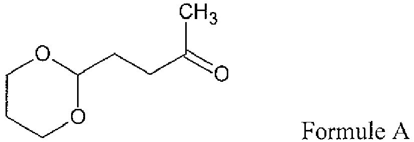

Dans la seconde voie, la première étape consiste en la formation d'un organomagnésien à partir du 2-(2-bromoéthyl)-1,3-dioxolane puis son addition sur le chlorure d'acétyle selon le schéma suivant : 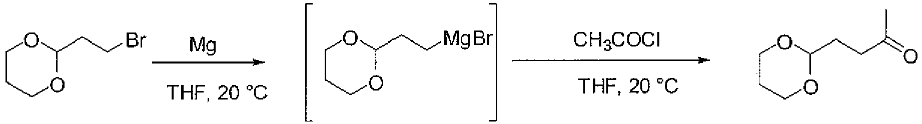 On obtient ainsi la 4-(1,3-dioxan-2-yl)butan-2-one de formule A suivante :

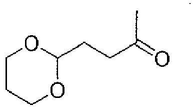

Cette cétone de formule A, a ensuite été engagée dans la formation du 2-[3-(cyclopenta-2,4-diénylidène)butyl]-1,3-dioxane, par action de la pyrrolidine et du cyclopentadiène lithié.

Le rendement maximum a été atteint en utilisant 1,1 équivalent de pyrrolidine ajouté environ 2 minutes avant l'addition de la solution de 1,5 équivalents de cyclopentadiène lithié dans le THF.

Le mélange est alors obtenu sous agitation environ une heure à température ambiante.

Le 2-[3-(cyclopenta-2,4-diénylidène)butyl]-1,3-dioxane de formule B suivante : 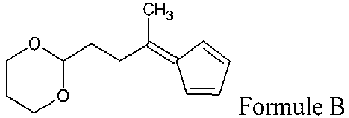 a été caractérisé en RMN ¹H par l'apparition d'un massif vers 6,5 ppm correspondant au cyclopentadiène.

En RMN ¹³C, le signal correspondant au carbone du groupe carbonyle (208,3 ppm) est déplacé à 143,2 ppm, et en spectroscopie IR une bande caractéristique des alcènes à 1639 cm⁻¹ apparaît.

Le fulvène obtenu est immédiatement mis en réaction dans l'étape suivante pour l'introduction d'un groupe méthyle sur le carbone 6 du fulvène afin d'obtenir le pseudo *tert*-butyle.

Cela a été effectué par action de méthyllithium sur le composé de formule B dans du diéthyl éther à -20 °C.

On obtient le 1-[4-(1,3-dioxan-2yl)-2-méthylbutyl]cyclopentadiényllithium de formule C suivante de façon quantitative :

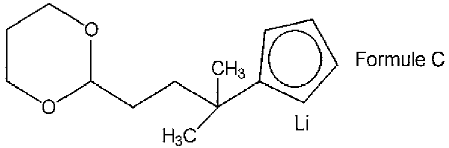

L'avancement de la réaction est facilement observable : la solution de fulvène est jaune vif et au fur et à mesure de la réaction, un précipité blanc apparaît et la solution se décolore pour devenir finalement incolore.

La filtration et le lavage sous argon du précipité obtenu permet d'obtenir le composé C sous forme d'une poudre blanche.

Ce dérivé cyclopentadiényllithium a été caractérisé en RMN ¹H par le blindage du massif formé par les protons du cyclopentadiène de 6,5 ppm à 5,6 ppm.

Le dérivé cyclopentadiényllithium de formule C a ensuite subi deux étapes de phosphination successives en ajoutant des quantités stoechiométriques de ClPPh₂ puis *n*-BuLi successivement.

On obtient alors le composé de formule D suivante avec un rendement global de 80 à 90 % sur les deux étapes de phosphination :

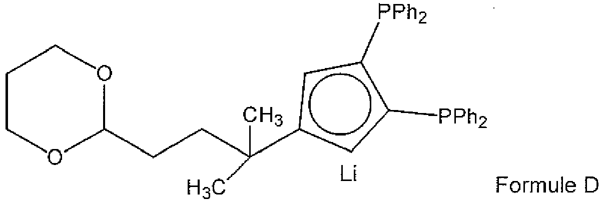

Ce composé a été caractérisé en RMN ¹H par la disparition de deux protons de l'anneau cyclopentadiényle ainsi que l'apparition d'un massif entre 7,0 et 7,5 ppm. La RMN ³¹P montre l'apparition d'un singulet à -25,2 ppm.

Ensuite, deux équivalents de lithien diphosphiné de formule D ont été mis à agir sur un équivalent de FeCl₂ dans le toluène ou le THF sous chauffage au reflux.

Ce composé obtenu a été purifié par chromatographie sur gel de silice.

On obtient le composé de formule (2) suivante sous la forme d'une poudre rouge avec 50% de rendement : le 1, 1', 2, 2'-tetrakis(diphénylphosphino)-4,4'-di[4-(1,3 dioxan-2-yl)-2-méthylbut-2-yl]ferrocène.

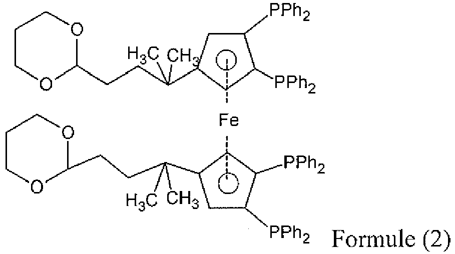

La synthèse de ce composé de formule (2) peut être également effectuée « one-pot » à partir du composé de formule C qui est un composé lithié mais non phosphiné ; en mettant à réagir le composé de formule C, en ajoutant dans une première étape, une quantité stoechiométrique de PPh₂Cl et *n*-BuLi à -80 °C puis à nouveau une quantité stoechiométrique PPh₂Cl et *n*-BuLi -80 °C suivi, toujours dans le même milieu réactionnel de l'ajout de 0,5 équivalent de FeCl₂ pour un équivalent de composé de formule C, dans du toluène sous chauffage au reflux.

Le produit obtenu est purifié par chromatographie sur gel de silice. Le rendement réactionnel est également de 50%.

Le composé de formule (2) a été caractérisé en RMN ¹H, ³¹P, ¹³C, masse exacte et diffraction des rayons X.

L'analyse en masse exacte et les spectres de RMN du proton et du carbone confirment la formation du dérivé ferrocénique cible.

Le spectre RMN ³¹P montre là encore des figures de couplage identiques au composé modèle : un système de spin de type AA'BB', comportant deux multiplets centrés sur -30,35 et -34,4 ppm, garant de la bonne conformation des atomes de phosphore et de leur proximité spatiale mutuelle.

La structure obtenue par diffraction X montre bien la conformation éclipsée des anneaux cyclopentadiène du ferrocène. On peut également voir qu'à l'état solide, les groupes cétal sont orientés dans des directions opposées.

La synthèse du composé de formule (3) consiste à libérer le groupement fonctionnel du composé de formule (2), afin d'avoir un ligand hétérogénéisable.

La déprotection d'aldéhydes ferrocénique n'est pas décrite précisément dans la littérature à la connaissance des inventeurs. Donc des essais de méthode de déprotection classique pour les composés de type 1,3-dioxane, telles que décrites dans « Protective groups in Organic Synthesis, Third Edition » par T. W. Greene et P. G. M. Wuts (1999 John Wiley & Sons) ont été menés.

Cependant, ces méthodes n'ont pas permis de déprotéger l'aldéhyde car ne menant jamais à une déprotection totale (75% au mieux).

Or, les inventeurs ont découvert de façon surprenante que l'utilisation de micro-ondes permettait de déprotéger les aldéhydes de manière quantitative.

L'appareillage utilisé permet de se placer sous pression supérieure à la pression atmosphérique et donc de pouvoir chauffer le mélange au dessus de son point d'ébullition en conditions normales.

Ainsi, en utilisant un rayonnement micro-ondes d'une puissance de 100 W pour 300 mg de composé et 15 mL de solvant à 120 °C, aux environs de 4 bars de pression dans le THF (THF/HCl 3N pendant 20 minutes), la déprotection s'avère être totale et le dialdéhyde est obtenu avec plus de 90% de rendement après purification par chromatographie sur gel de silice. On obtient alors le composé de formule (3) : le 1, 1', 2, 2'-tetrakis(diphénylphosphino)-4,4'-di(4-oxo-2-méthylbut-2-yl)ferrocène.

Ce composé est caractérisé en RMN ¹H par la disparition des signaux dus aux groupements protecteurs (proton du cétal à 4,47 ppm) et par l'apparition du signal correspondant au proton aldéhydique à 9,77 ppm. De même en RMN ¹³C, les signaux du cétal n'apparaissent plus et un signal à 202,0 ppm typique des carbonyles est apparu. En spectroscopie IR, on observe une bande à 1721,8 cm⁻¹.

Le spectre RMN ³¹P possède toujours le même aspect de système de spin AA'BB' mais les déplacements chimiques des deux multiplets ont légèrement changé : ils sont blindés d'environ 0,4-0,5 ppm à -30,9 et -34,9 ppm.

### Exemple 3 : Immobilisation du composé de formule (3)

Cette immobilisation a été effectuée par greffage sur une résine polystyrène-méthylaminée indexée PL-AMS.

Pour cela, une réaction de condensation entre la résine polystyrène-méthylaminée et le composé-ligand hétérogénéisable de formule (3) a été effectuée par amination réductrice dans le 1,2-dichloroéthane en présence de triacétoxyborohydrure de sodium.

Cet agent réducteur est spécifique des imines et présente l'avantage de ne pas engendrer de sous-produits potentiellement toxiques comme peut le faire NaBH₃CN. La résine a été mise en suspension 30 minutes avant l'ajout des réactifs afin de la faire gonfler et permettre l'accès aux sites réactionnels à l'intérieur des billes. Après 24 heures de réaction, le milieu réactionnel a été neutralisé par une solution aqueuse 1 M de NaOH, puis la résine a été lavé abondamment par du CH₂Cl₂ pour éliminer le produit n'ayant pas réagi.

La résine obtenue est purement hétérogène, elle n'est soluble dans aucun solvant. Aussi pour vérifier l'intégrité du ligand, une analyse de RMN CP-MAS ³¹P a été effectuée.

Le spectre a été obtenu à une vitesse de rotation de 14 kHz et révèle la présence d'un signal centré sur -30 ppm. Ce déplacement est concordant avec le déplacement observé pour le ligand correspondant en solution et montre que les phosphines n'ont pas été altérées lors de leur greffage sur la résine, en particulier les atomes de phosphore ne sont pas oxydés comme le montre ce déplacement chimique en RMN

### Exemple 4 : Synthèse du composé de formule (4)

Pour obtenir le composé de formule (4), on a procédé à une réduction du composé de formule (3).

La réduction du carbonyle a été réalisée par LiAlH₄ à 0 °C dans le THF.

La réaction est complète après 1 heure et ne nécessite aucune purification après le traitement classique du milieu réactionnel ; elle donne quantitativement le composé de formule (4), c'est-à-dire le 1, 1', 2, 2'-tetrakis(diphénylphosphino)-4,4'-di(4-hydroxy-2-méthylbut-2-yl)ferrocène caractérisé par la disparition en RMN ¹H du signal à 9,77 ppm et l'apparition d'un signal correspondant aux protons du groupement méthylène en a de l'oxygène (3,62 ppm). De même, en RMN ¹³C, le signal du carbone du carbonyle à 202,0 ppm est déplacé vers les champs forts à 63,6 ppm. Le spectre de RMN ³¹P reste inchangé.

### Exemple 5 : Synthèse du composé de formule 5

### Méthode A :

A une solution de iodure de triphényl(4-vinylbenzyl)phosphonium (0,68 g, 1,33 mmol, 3 equiv.) dans 15 ml de THF est ajouté à température ambiante (t. a.) du NaH (32 mg, 1,35 mmol, 3 equiv.). Le composé (3) (0,5 g, 0,45 mmol, 1 equiv.) en solution dans 5 ml de THF est additionné goutte à goutte sur la solution de phosphonium. Après 30 min à t. a. le milieu réactionnel est neutralisé par une solution aqueuse saturée de NH₄Cl, puis extrait par 2 fois par 20 ml de CH₂Cl₂. Après un lavage avec une solution aqueuse saturée de NaCl, la phase organique est séchée sur MgSO₄ puis concentrée sous pression réduite pour donner après purification par chromatographie sur gel de silice (AcOEt/hexane 1:4) 300 mg (ρ = 50%) de 1,1',2,2'-tétrakis(diphénylphosphino)-4,4'-bis[2-méthyl-6-(4-vinylphényl)hex-5-èn-2-yl] ferrocène sous forme d'une poudre rouge.

### Méthode B :

A une solution du composé (3) (0,1 g, 89 µmol, 1 equiv.) dans 1,5 ml de THF est ajouté le 2-(4-vinylbenzylsulfonyl)benzo[d]thiazole (0,06 g, 0,19 mmol, 2,1 mmol) puis du NaHMDS (0,1 ml, 2 M dans THF, 2,1 equiv.) à t. a. Après 30 min à t. a. le milieu réactionnel est neutralisé par une solution aqueuse saturée de NH₄Cl, extrait par 2×20 ml de CH₂Cl₂. Après un lavage avec une solution aqueuse saturée de NaCl, la phase organique est séchée sur MgSO₄ puis concentrée sous pression réduite pour donner après purification par chromatographie sur gel de silice (AcOEt/hexane 1:4) 41 mg (ρ = 35%) de 1,1',2,2'-tétrakis(diphénylphosphino)-4,4'-bis[2-méthyl-6-(4-vinylphényl)hex-5-èn-2-yl] ferrocène sous forme d'une poudre rouge. Le spectre RMN ³¹P conserve comme attendu l'aspect de système de spin AA'BB' avec des déplacements chimiques légèrement modifiés. **RMN ¹H** (CDCl₃, 600 MHz, 298 K): δ(ppm) 6,41-8,44 (m, 50H), 6,42 (d, 2H, ³J = 16,8 Hz), 6,22 (m, 2H), 5,77 (d, 2H, ³J = 17,4 Hz), 5,25 (d, 2H, ³J = 11,4 Hz), 4,10, 4,23 (s, 2H), 1,94, 2,12 (m, 2H chacun), 1,28, 1,38 (m, 2H chacun), 0,18, 1,07 (s, 6H chacun). **RMN ³¹P** (CDCl₃, 121,48 MHz, 298 K) : δ(ppm) -30,8 (m, 2P), -34,9 (m, 2P). **RMN ¹³C** (CDCl₃, 150,9 MHz, 298 K): δ(ppm) 126,1-139,0 (m, 60C), 136,5 (s, 2C), 131,2 (s, 2C), 129,4 (s, 2C), 113,4 (s, 2C), 105,5 (s, 2C), 88,1 (m, 2C), 79,7 (m, 2C), 72,6, 72,1 (s, 2C chacun), 46,7 (s, 2C), 33,3 (s, 2C), 28,8 (s, 2C), 28,6, 27,1 (s, 2C chacun). IR υ(cm⁻¹) : 1476,2, 1433,2. C₈₈H₈₃P₄Fe (MW 1319,33, masse exacte 1318,47): *m*/*z* 1319,483 (M+); simulé 1319,479 (*σ* = 0,083).

### Exemple 6 : Synthèse du composé de formule (6)

Le composé de formule (6) a été obtenu par amination réductrice du composé (3) avec l'amine de formule suivante : 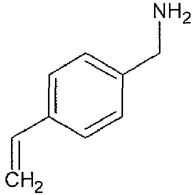 par une réaction de Staudinger selon la méthode décrite par Ahuji et al., J. Polym. Sci. Pol. Chem. 2005, 43, 3411.

L'amination réductrice est réalisée dans le dichloroéthane en présence de trois équivalents de triacétoxyborohydrure de sodium et conduit après purification par chromatographie au composé de formule (6) avec 55% de rendement.

Le composé de formule (6), c'est-à-dire le 1, 1', 2, 2'-tetrakis(diphénylphosphino)-4,4'-[2-méthyl-5-(4-vinylbenzylamino)pent-2-yl)ferrocène a été caractérisé par spectrométrie de masse exacte avec la détection d'une massif centré sur m/z = 1353,353. En RMN ¹H, on observe la disparition du signal à 9,77 ppm et l'apparition de signaux typiques d'un système de spin de type vinylique entre 5 et 6 ppm.

De même sur le spectre de RMN ¹³C, le signal correspondant au carbonyle à 202,0 ppm disparaît et on voit apparaître des signaux de carbone sp² à 136,8 et 113,8 ppm ainsi que le signal du carbone benzylique à 53,4 ppm. Le spectre RMN ³¹P possède toujours le même aspect de système de spin AA'BB'.

### Exemple 7 : Immobilisation du composé de formule (6) par polymérisation

Le composé de formule (6) a été immobilisé par co-polymérisation avec un monomère de formule suivante :

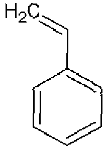

Dans un ballon de 100 mL sont additionnés 1,54 g de styrène (14,8 mmol) et 0,260 g de phosphine ferrocénique (0,2 mmol) dans 50 mL de toluène. Le mélange est dégazé par barbotage d'azote pendant 1 heure puis 0,04 g d'azobisisobutyronitrile (AIBN) comme agent initiateur de polymérisation radicalaire sont ajoutés.

Puis, le mélange réactionnel est maintenu à 85 °C sous agitation magnétique pendant 48 heures. A l'issue de ce temps, le toluène est évaporé, et un minimum de THF est ajouté pour solubiliser le résidu polymèrique.

La solution de THF est ajoutée goutte à goutte à 50 mL de méthanol refroidit à 0 °C par un bain de glace. Le polymère insoluble dans le méthanol précipite lentement.

A la fin de l'addition le polymère en suspension dans le méthanol est filtré sur verre fritté, rincé avec un minimum de méthanol, et séché sous vide pour une masse finale de 1,14 g.

Le composé attendu répond à la formule suivante :

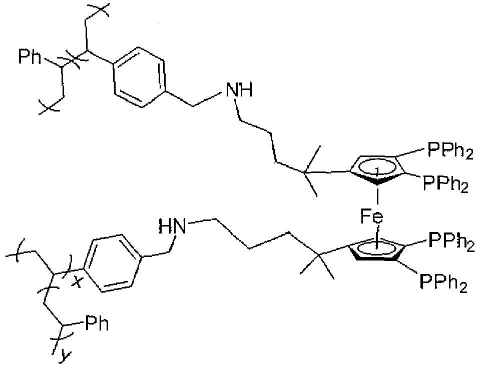

La RMN du phosphore en solution confirme que ce composé-ligand supporté est obtenu avec le contrôle conformationnel du ligand de formule (6) comme démontré par les deux multiplets centrés à -34,30 et -30,30 ppm en RMN du phosphore en solution dans le chloroforme deutérié (système de spin AA'BB').

Le polymère obtenu présente une forte coloration rouge et est soluble dans des solvants tels que le THF, le CHCl₃, CH₂Cl₂, toluène, Et₂O et acétate d'éthyle alors qu'il est insoluble dans l'hexane et le méthanol. L'analyse élémentaire de ce polymère a révélé une teneur en phosphore de 1,05% ce qui correspond à une charge en ligand de 0,085 mmol/g.

Les performances catalytiques des ligands supportés selon l'invention ont été prouvées en catalyse de couplage carbone-carbone.

L'exemple 8 suivant montre ces performances catalytiques.

### Exemple 8 : réaction de couplage d'arylation de l'acide phényle boronique au composé désactivé 4-methoxybromobenzène par le composé de formule (6) supporté sur résine polystyrène, et récupération du catalyseur.

Cette réaction de couplage carbone-carbone a été réalisée dans les conditions de basses charges métalliques (<1%, conditions réactionnelles non optimisées) suivantes : un mélange solide d'acide phényle boronique (0,26 g, 2,13 mmol) de carbonate de potassium (0,30 g, 2,17 mmol), du composé-ligand supporté polymérique issu du composé (6) (0,125 g de résine polymère à 0,085 mmol/g de ligand tétraphosphine, soit 0,01065 mmol) et du précurseur de palladium [Pd(C₃H₅)Cl]₂ (1,9 mg, 0,0052 mmol) est placé dans un tube de Schlenk sous argon. 1 mL de DMF distillé et dégazé par barbotage à l'azote est additionné et le mélange est maintenu à 80 °C sous forte agitation pendant cinq minutes. La 4-bromoanisole (0,41 g, 265 µL, 2,17 mmol) distillée et dégazée est ajoutée par seringue au mélange réactionnel qui est maintenu pour 20 h à 120 °C. Le mélange refroidi est évaporé à sec sous vide, et le résidu solubilisé dans 10 mL d'éther éthylique et extrait avec 10 mL d'eau. Les phases organiques sont séchées sur sulfate de magnésium et après évaporation de l'éther sous-vide, le résidu est mis en solution dans 30 mL de méthanol pour séparer la résine complexée au palladium (catalyseur). Le précipité résultant (catalyseur) est filtré, et le produit de couplage est obtenu avec 50% de rendement après évaporation du solvant et traitement chromatographique.

Il faut signaler que la grande majorité des couplages de ce type utilisant des résines dans la bibliographie est réalisée à partir de iodures et en présence de charges métalliques dépassant 1 mol% (N. T. S. Phan et al. Adv. Synth. Catal. 2006, 348, 609-679, voir p. 636-650 et références citées). Ici le 4-methoxybromobenzène est reconnu comme un substrat difficile à coupler car riche électroniquement (voir J.-C. Hierso et al. Eur. J. Inorg. Chem. 2007, 3760-3780). En l'absence d'un ligand, Buchwald et co-auteurs ont rapporté la déficience de couplage en présence de palladium acétate, alors que la 4-bromoacétophenone (substrat activé, pauvre en électron) est bien couplée à l'acide phényle boronique (voir Buchwald et al. Ang. Chem. Int. Ed. 1999, 38, 2413-2416, et J. Am. Chem. Soc. 1999, 121, 9550-9561).

### Exemple 9: réaction de couplage d'arylation de l'acide phényle boronique au bromobenzène par le composé de formule (6) supporté sur résine polystyrène, et récupération du catalyseur.

Cette réaction de couplage carbone-carbone a été réalisée dans les conditions de basses charges métalliques < 0,5 mol % suivantes : un mélange solide d'acide phényle boronique (0,26 g, 2,13 mmol) de carbonate de potassium (0,30 g, 2,17 mmol), du composé-ligand supporté polymérique issu du composé (6) (0,125 g de résine polymère à 0,085 mmol/g de ligand tétraphosphine, soit 0,01065 mmol) et du précurseur de palladium [Pd(C₃H₅)Cl]₂ (1,9 mg, 0,0052 mmol) est placé dans un tube de Schlenk sous argon. 1 mL de DMF distillé et dégazé par barbotage à l'azote est additionné et le mélange est maintenu à 80 °C sous forte agitation pendant cinq minutes. Le bromobenzène (0,34 g, 230 µL, 2,17 mmol) distillé et dégazé est ajouté par seringue au mélange réactionnel qui est maintenu pour 20 h à 120 °C. Le mélange refroidi est évaporé à sec sous vide, et le résidu solubilisé dans 10 mL d'éther éthylique et extrait avec 10 mL d'eau. Les phases organiques sont séchées sur sulfate de magnésium et après évaporation de l'éther sous-vide, le résidu est mis en solution dans 20 mL de méthanol pour séparer la résine complexée au palladium (catalyseur). Le précipité résultant (catalyseur) est filtré, et l'analyse de la solution par GC indique une conversion totale du bromobenzène en produit de couplage, obtenu avec 80% de rendement isolé après séparation du catalyseur hétérogène, puis évaporation du solvant organique contenant le produit et traitement chromatographique. Le catalyseur isolé par filtration est réengagé en réaction selon la même procédure. L'analyse GC montre a nouveau une conversion totale du bromobenzène en produit de couplage, obtenu avec 80% de rendement après évaporation du solvant et traitement chromatographique. Le catalyseur isolé peut ainsi être réengagé plusieurs fois (six fois) sans baisse d'activité notable, la limitation résidant dans les pertes lors de la filtration.

### Exemple 10 : réaction de couplage d'arylation de l'acide phényle boronique à la bromoacétophénone par le composé de formule (6) supporté sur résine polystyrène, et récupération du catalyseur.

Cette réaction de couplage carbone-carbone a été réalisée dans les conditions de basses charges métalliques < 0.5 mol% suivantes : un mélange solide d'acide phényle boronique (0,26 g, 2,13 mmol) de carbonate de potassium (0,30 g, 2,17 mmol), de bromoacétophénone (0,43 g, 2,17 mmol), du composé-ligand supporté polymérique issu du composé (6) (0,125 g de résine polymère à 0,085 mmol/g de ligand tétraphosphine, soit 0,01065 mmol) et du précurseur de palladium [Pd(C₃H₅)Cl]₂ (1,9 mg, 0,0052 mmol) est placé dans un tube de Schlenk sous argon. 1 mL de DMF distillé et dégazé par barbotage à l'azote est additionné et le mélange est maintenu pour 20 h à 120 °C. Le mélange refroidi est évaporé à sec sous vide, et le résidu solubilisé dans 10 mL d'éther éthylique et extrait avec 10 mL d'eau. Les phases organiques sont séchées sur sulfate de magnésium et après évaporation de l'éther sous-vide, le résidu est mis en solution dans 20 mL de méthanol pour séparer la résine complexée au palladium (catalyseur). Le précipité résultant catalyseur est filtré, et l'analyse de la solution par GC indique une conversion totale de la bromoacétophénone en produit de couplage, isolé avec 65% de rendement après évaporation du solvant et traitement chromatographique. Le catalyseur isolé par filtration est réengagé en réaction selon la même procédure. L'analyse GC montre a nouveau une conversion totale de la bromoacétophénone en produit de couplage, obtenu avec 65% de rendement après évaporation du solvant et traitement chromatographique. Le catalyseur isolé peut ainsi être réengagé plusieurs fois (six fois) sans baisse d'activité notable, la limitation résidant dans les pertes lors de la filtration.

### Exemple 11 : Synthèse du composé de formule 7.

Le composé de formule (4) (50 mg, 44 µmol) est solubilisé dans 1 ml de dichlorométhane. Le (3-isocyanatopropyl)triethoxysilane (24 µl, 90 µmol) est introduit dans le milieu réactionnel puis une goutte de triéthylamine est ajoutée. Après 20 h à température ambiante, le milieu est concentré sous vide pour donner 70 mg en rendement de 95% du produit sous forme d'une poudre rouge. Le spectre RMN ³¹P conserve comme attendu l'aspect de système de spin AA'BB' avec des déplacements chimiques légèrement modifiés. **RMN ¹H** (CDCl₃, 500 MHz, 298 K): δ(ppm) 6,45-8,42 (m, 40H), 4,7 (s élargi, 2H), 4,17-4,04 (s, 2H chacun), 4,02 (t, 4H, ³*J* = 6,5 Hz), 3,82 (q, 12H, ³*J* = 7 Hz), 3,20 (q, 4H, ³*J* = 6,5 Hz), 1,65 (m, 4H), 1,30 (m, 4H), 1,24 (t, 18H, ³*J* = 7 Hz), 0,99, 0,13 (s, 6H chacun), 0,87 (m, 4H), 0,63 (s, 4H). **RMN ³¹P** (CDCl₃, 202,5MHz, 298 K) : δ(ppm) -30,7 (m, 2P), -34,6 (m, 2P). **RMN ¹³C** (CDCl₃, 125,7 MHz, 298 K): δ(ppm) 157,1 (s, 2C), 127,5-139,1 (m, 48C,), 105,8 (s, 2C), 88,5 (dd, 2C, ¹*J*_{CP} = 35,2Hz, ²*J*_{CP} = 13,8Hz), 79,9 (t, 2C, *J*_{CP} = 18,9Hz), 72,7, 72,2 (s, 2C chacun), 65,6 (s, 2C), 58,8 (s, 6C), 43,79 (s, 2C), 43,3 (s, 2C), 35,7 (s, 2C), 28,9, 27,2 (s, 2C chacun), 25,1 (s, 2C), 23,7 (s, 2C), 18,6 (s, 2C), 8,0 (s, 2C). C₉₀H₁₁₂P₄FeO₁₀N₂Si₂ (MW 1617,77, masse exacte 1616,61): *m*/*z* 1616,613 M⁺; simulé 1616,615 (σ = 0,189).

### Exemple 12 : Immobilisation du composé de formule 7 sur support inorganique SiO₂.

1 g de silice (ACROS, Kieselgel 0,035-0,070mm, 60Å) est placée en suspension dans du toluène dans un appareil de Dean-Stark et chauffée au reflux pendant 20 h. Le composé disilane (7) (300 mg, 185 µmol) en solution dans 15 ml de toluène est additionné sur la silice et le mélange réactionnel est placé 5 h au reflux. Après refroidissement, le milieu est concentré sous vide puis repris dans 20 ml de dichlorométhane, filtré, lavé abondamment par du dichlorométhane puis séché sous vide pour donner 1,06 g d'une poudre rouge. Le solide obtenu est insoluble dans tous les solvants organiques usuels testés (THF, CHCl₃, CH₂Cl₂, toluène, Et₂O, acétate d'éthyle, hexane, méthanol, DMF, DMSO, NMP, etc.). L'analyse RMN CP-MAS ³¹P obtenu à une vitesse de rotation de 14 Hz révèle la présence d'un signal centré sur -30 ppm. Ce déplacement est concordant avec le déplacement observé pour le ligand correspondant en solution.

### Exemple 13 : réaction de couplage d'arylation de l'acide phényle boronique à la bromoacétophénone par le composé de formule (7) supporté sur support inorganique SiO₂ et récupération du catalyseur.

Cette réaction de couplage carbone-carbone a été réalisée dans les conditions de basses charges métalliques < 0.5 mol% suivantes : un mélange solide d'acide phényle boronique (0,26 g, 2,13 mmol) de carbonate de potassium (0,30 g, 2,17 mmol), de bromoacétophénone (0,43 g, 2,17 mmol), du composé-ligand supporté sur silice issu du composé (7) (0,15 g de résine polymère à 0,068 mmol/g de ligand tétraphosphine, soit 0,0102 mmol) et du précurseur de palladium [Pd(C₃H₅)Cl]₂ (1,8 mg, 0,00549 mmol) est placé dans un tube de Schlenk sous argon. 1 mL de DMF distillé et dégazé par barbotage à l'azote est additionné et le mélange est maintenu pour 20 h à 120 °C. Le mélange refroidi est filtré pour récupérer le composé-ligand et le métal (moins de 1 ppb de Pd analysé par ICP restant en solution). Le filtrat est évaporé à sec sous vide, et le résidu solubilisé dans 10 mL d'éther éthylique avec 10 mL d'eau pour extraction. Les phases organiques sont séchées sur sulfate de magnésium. L'analyse par GC préalable indique une conversion totale de la bromoacétophénone en produit de couplage, pour 85% de rendement après évaporation du solvant et traitement chromatographique. Le catalyseur isolé par filtration est réengagé en réaction selon la même procédure. L'analyse GC montre a nouveau une conversion totale de la bromoacétophénone en produit de couplage, obtenu avec 85% de rendement après évaporation du solvant et traitement chromatographique. Le catalyseur isolé peut ainsi être réengagé plusieurs fois (six fois) sans baisse d'activité notable, la limitation résidant dans les pertes lors de la filtration.

### Exemple 14 : Synthèse du composé de formule 8.

A une solution du composé de formule (3) (0,5g, 0,41mmol) dans 20 ml de dichlorométhane est ajouté à température ambiante du 3-amino(triéthoxypropyl)silane (0,19ml, 0,82 mmol) et une goutte d'acide acétique. La solution est portée au reflux 5 h puis concentrée sous vide. Le brut est purifié par chromatographie sur gel de silice (éluant CH₂Cl₂) pour donner le composé de formule (8) sous forme d'une poudre orange (75% de rendement). Le spectre RMN ³¹P conserve comme attendu l'aspect de système de spin AA'BB' avec des déplacements chimiques légèrement modifiés. **RMN ¹H** (CDCl₃, 500 MHz, 298 K): δ(ppm) 6,45-8,42 (m, 40H), 7,50 (t, 2H, ³*J* = 5 Hz), 4,17-4,04 (s, 2H chacun), 3,82 (q, 12H, ³*J* = 7 Hz), 1,63 (t, 4H, ³*J* = 6,5 Hz), 1,56 (q, 4H, ³*J* = 6,5 Hz), 1,35 (m, 4H), 1,30 (m, 4H), 1,24 (t, 18H, ³*J* = 7 Hz), 0,99, 0,13 (s, 6H chacun), 0,63 (s, 4H). **RMN ³¹P** (CDCl₃, 202,5MHz, 298 K) : δ(ppm) -30,8 (m, 2P), -34,3 (m, 2P). L'immobilisation du composé de formule (8) sur support inorganique SiO₂ est réalisée de la même manière que pour le composé (7).

### Exemple 15 : Synthèse du composé de formule 9 avec E = (CH₂)₃ et GF = 4-vinylbenzylamine.

A une suspension de FeCl₂ (1,1 g, 8,6 mmol) dans du toluène (20 ml) est ajoutée goutte à goutte à -40 °C une solution de 1,2-bis(diphénylphosphino)-4-*tert-*butylcyclopentadiényle lithium (4,45 g, 8,9 mmol) dans 30 ml de toluène. La solution est maintenue sous agitation deux heures après retour à température ambiante. Une solution de 1,2-bis(diphénylphosphino)-3-[4-(1,3-dioxan-2-yl)-2-méthylbutyl]cyclopentadiényle lithium (5,07 g, 8,55 mmol) dans 30 ml de toluène est ensuite additionnée goutte à goutte à -40 °C. Le mélange est porté au reflux 15 h. Après retour à température ambiante, la solution est lavée par 50 ml d'eau, puis 50 ml d'une solution aqueuse d'HCl 0,5 M. La phase aqueuse est extraite par 50 ml de toluène, puis les phases organiques sont rassemblées, séchées sur MgSO₄ et concentrées sous pression réduite. Le brut obtenu est purifié par chromatographie sur gel de silice (AcOEt/heptane, 1 :5) pour donner 1 g (ρ = 11%) de 1,1',2,2'-tetrakis(diphénylphosphino)-4,4'-di*tert*-butylferrocène, 3 g (ρ = 32%) de 1,1',2,2'-tétrakis(diphénylphosphino)-4-[4-(1,3-dioxan-2-yl)-2-méthylbut-2-yl]-4'-tert-butylferrocène et 2 g (p = 22%) de 1,1',2,2'-tétrakis(diphényl phosphino)-4,4'-di[4-(1,3-dioxan-2-yl)-2-méthylbut-2-yl]ferrocène recherché. Contrairement aux composés de formule (1) à (8), le spectre RMN ³¹P des trois produits possède du fait de la dissymétrie de la molécule, un spectre de type ABCD avec des déplacements chimiques différents pour les quatre atomes de phosphore. Ceux-ci ne peuvent toutefois pas être extraits directement du spectre mais doivent être calculés par simulation (G-NMR). **RMN ¹H** (CDCl₃, 300 MHz, 303 K): δ(ppm) 6,30-8,42 (m, 40H), 4,34 (m, 1H), 3,88-4,05 (m, 6H), 3,69 (m, 2H), 2,01 (m, 1H), 1,21-1,51 (m, 5H), 0,84 (s, 3H), 0,6 (s, 9H), 0,05 (s, 3H). **RMN ³¹P** (CDCl₃, 121,49 MHz, 303 K) : δ(ppm) -30,0 (m, 2P), -33,9 (m, 2P). Valeurs calculées: -29,9, -30,3, -33,9, -34,0 ppm. **RMN ¹³C** (CDCl₃, 75,46 MHz, 303 K): δ(ppm) 127,4-139,4 (m, 48C), 107,7 (s, 1C), 105,7 (s, 2C), 103,1 (s, 2C), 88,3 (m, 2C), 79,7 (m, 2C), 73, 72,5, (m, 2C chacun), 72,0, 71,2 (d, 2C chacun, *J*_{CP} = 5,3 Hz), 67,3, 61,2 (s, 1C chacun), 41 (s, 1C), 33,2 (s, 1C), 31,9 (s, 3C), 31,1, 30,7 (s, 1C chacun), 28,9, 27,3 (s, 1C chacun), 26,3 (s, 2C).C₇₁H₇₁P₄FeO2 (MW 1135,05, masse exacte 1135,36): *m*/*z* 1135,3767 M⁺; simulé 1135,3751 (*σ* = 0,035).
Pour la déprotection amenant au composé de formule (9) proprement dite, avec E = (CH₂)₃ et GF = 4-vinylbenzylamine, une solution de 1,1',2,2'-tétrakis(diphénylphosphino)-4-[4-(1,3-dioxan-2-yl)-2-méthylbut-2-yl]-4'-tert-butylferrocène (0,1 g, 88 µmol) dans 20 ml de THF sont ajoutés 5 ml d'une solution d'acide chlorhydrique 2 N. La solution est placée sous rayonnement micro-onde (125 W) selon le programme suivant : montée en température 10 °C/min jusqu'à 120 °C, puis plateau à 120 °C pendant 10 min. Après refroidissement, le milieu réactionnel est concentré sous vide, dilué avec 20 ml d'eau et extrait par 2 fois 25 ml de CH₂Cl₂. La phase organique est séchée sur MgSO₄, et concentrée sous pression réduite. Le brut obtenu est purifié par chromatographie sur gel de silice (éluant AcOEt/heptane 1:4) pour donner 90 mg (p = 90%) de 1,1',2,2'-tétrakis(diphénylphosphino)-4-(4-oxo-2-méthylbut-2-yl)-4'-tert-butylferrocène sous forme d'une poudre rouge pour laquelle le spectre RMN ³¹P conserve comme attendu l'aspect de système de spin ABCD avec des déplacements chimiques légèrement modifiés.

En dernière étape, une solution de NaBH(OAc)₃ (0,39 g, 1,8 mmol) et de 4-vinylbenzylamine (0,3 g, 2,3 mmol) dans 5 ml de dichloroéthane est ajoutée goutte à goutte à température ambiante une solution de 1,1',2,2'-tétrakis(diphénylphosphino)-4-(4-oxo-2-méthylbut-2-yl)-4'-tert-butylferrocène (1,3 g, 1,2 mmol) dans 15 ml de dichloroéthane. La solution est maintenue sous agitation 24 h puis neutralisée par ajout d'une solution aqueuse de soude 1 M. La phase aqueuse est extraite par 2 fois 30 ml de toluène. Les phases organiques sont rassemblées, séchées sur MgSO₄ et concentrées sous pression réduite. Le brut obtenu est purifié par chromatographie sur gel de silice (CH₂Cl₂/MeOH, 95:5) pour donner 0,92 g (ρ = 64%) du composé de formule (9) sous forme d'une poudre rouge-orange. Le spectre RMN ³¹P conserve comme attendu l'aspect de système de spin ABCD avec des déplacements chimiques légèrement modifiés. **RMN ¹H** (CDCl₃, 500MHz, 300 K): δ(ppm) 6,45-8,42 (m, 45H), 5,75 (dd, 1H, ³*J* = 10,5 Hz, ²*J* > 1 Hz), 5,25 (dd, 1H, ³*J* = 6,5 Hz, ²*J* >1 Hz) 4,18, 4,16, 4,08, 4,02 (s, 1H chacun), 3,83 (s, 2H), 2,60 (t, 2H, ³*J* = 3,5 Hz), 1,21-1,51 (m, 4H), 0,98, 0,15 (s, 3H chacun), 0,71 (s, 9H). **RMN ³¹P** (CDCl₃, 202,45MHz, 300 K) : δ(ppm) -30,2 (m, 2P), -33,8 (m, 2P). Valeurs calculées : -29,9, -30,4, -33,7, -33,9 ppm. **RMN ¹³C** (CDCl₃, 125,75 MHz, 300 K): δ(ppm) 140, 6 (s, 1C), 137 (s, 2C), 136,8 (s, 1C),128,7 (s, 2C), 127,1-137,5 (m, 48C), 126,4 (s, 1C), 113,9 (s, 1C), 107,7, 106,2 (s, 1C chacun),88,2 (m, 2C), 79,7 (m, 2C), 72,8, 72,6, 71,9, 71,3 (s, 1C chacun), 54,4 (s, 1C), 50,7 (s, 1C), 44,9 (s, 1C), 33,41, 30,7 (s, 1C chacun), 31,9 (s, 1C), 28,8, 27,5 (s, 1C chacun), 26,1 (s, 1C). C₇₇H₇₆P₄FeN (MW 1238,32, masse exacte 1137,48): *m*/*z* 1194,425 (M+Na)⁺; simulé 1194,428 (σ = 0,048).

### Exemple 16 : Synthèse du composé de formule 10 avec E = (CH₂)₃ et GF = 4-vinylbenzylamine.

A une suspension de FeCl₂ (0,45 g, 3,54 mmol, 1 equiv.) dans du THF (20 ml) est ajoutée à -80 °C une solution de 1,2-bis(diphénylphosphino)-4-tert-butylcyclopentadiényle lithium (2,08 g, 3,48 mmol, 1 equiv.) dans 30 ml de THF. La solution, maintenue sous agitation 1 h après retour à température ambiante, devient rouge foncé. Une solution de di*iso*propylphosphinocyclopentadiényle lithium (0,65 g, 3,45 mmol, 1 equiv.) dans 20 ml de THF est ajoutée à -80 °C. Après retour à température ambiante, la solution rouge foncé est portée au reflux pour 4 h. La solution est filtrée sur silice, concentrée sous pression réduite et purifiée par chromatographie sur gel de silice (éluant AcOEt/heptane 1:3) pour donner 800 mg (ρ = 30%) de 1,2-bis(diphénylphosphino)-1'-di*iso*propylphosphino-4-[4-(1,3-dioxan-2-yl)-2-méthylbut-2-yl]-butylferrocène sous forme d'une poudre orange.
A une solution de 1,2-bis(diphénylphosphino)-1'-di*iso*propylphosphino-4-[4-(1,3-dioxan-2-yl)-2-méthylbut-2-yl]-butylferrocène (1 g, 1,2 mmol) solubilisé dans 100 ml de THF est ajouté 15 ml d'une solution d'acide chlorhydrique 2 N. Après 2 heures sous agitation à reflux le milieu réactionnel est neutralisé par une solution aqueuse saturée d'hydrogénocarbonate de sodium et extrait par 2x50 ml de CH₂Cl₂. La phase organique est séchée sur MgSO₄, et concentrée à l'évaporateur rotatif. Le brut obtenu est purifié par chromatographie sur gel de silice (éluant AcOEt/heptane 1:4) pour donner 630 mg (p = 67%) de 1,2-bis(diphénylphosphino)-1'-di*iso*propylphosphino-4-(4-oxo-2-méthylbut-2-yl)ferrocène sous forme d'une poudre rouge.
A une solution de NaBH(OAc)₃ (0,17 g, 0,8 mmol) et de 4-vinylbenzylamine (0,26 g, 1,95 mmol) dans 10 ml de dichloroéthane est ajoutée goutte à goutte à température ambiante une solution de 1,2-bis(diphénylphosphino)-1'-di*iso*propylphosphino-4-(4-oxo-2-méthylbut-2-yl)ferrocène (1,3 g, 1,2 mmol) dans 5 ml de dichloroéthane. La solution est maintenue sous agitation 24 h puis neutralisée par ajout d'une solution aqueuse de soude 1M. La phase aqueuse est extraite par 2 fois 30 ml de dichlorométhane. Les phases organiques sont rassemblées, séchées sur MgSO₄ et concentrées sous pression réduite. Le brut obtenu est purifié par chromatographie sur gel de silice (CH₂Cl₂/MeOH, 95:5) pour donner 0,4 g (ρ = 55 %) de 1,1',2-tris(diphénylphosphino)-2'-di*iso*propylphosphino-4-[2-méthyl-5-(4-vinyl benzylamino)pent-2-yl]ferrocène sous forme d'une poudre rouge-orange. Le spectre RMN ³¹P conserve, comme attendu, l'aspect de système de spin A₂B typique des ces triphosphines, comme décrit par Hierso *et al.* pour l'analogue non-hétérogénéisables dans Org. Lett. 2004, 6, 3473-3476, avec des déplacements chimiques légèrement modifiés. **RMN ¹H** (CDCl₃, 300 MHz, 303 K): δ(ppm) 6,85-7,70 (m, 24H), 6,69 (dd, 1H, ³*J* = 17,7 Hz, ³*J* = 10,8 Hz), 5,73 (dd, 1H, ³*J* = 17,7 Hz, ²*J* = 1,5 Hz), 5,24 (dd, 1H, ³*J* = 10,8 Hz, ²*J* = 1,5 Hz), 4,11 (m, 4H), 3,89 (m, 4H), 2,75 (m, 2H), 1,58 (m, 4H), 1,45 (hd, 2H, ³*J_{HH}* = 6,9 Hz, ²*J_{HP}* = 2,9 Hz), 1,30 (s, 6H), 0,84 (dd, 6H, ³*J_{HP}* = 12,9 Hz, *³J_{HH}* = 6,9 Hz) 0,60 (s, 6H, ³*J_{HP}* = 12,9 Hz, ³*J_{HH}* = 6,9 Hz). **RMN ³¹P** (CDCl₃, 121,49MHz, 303 K) : δ(ppm) -3,4 (s, 1P), -25 (s, 2P). **RMN ¹³C** (CDCl₃, 75MHz, 298 K): δ(ppm) 127,5-139 (m, 24C), 136,3 (s, 1C), 127,5 (s, 2C), 126,5 (s, 2C), 114,2 (s, 1C), 106,4 (s, 1C), 81,5 (d, 1C, ¹*J*_{CP} = 21Hz,), 81,5 (s, 1C), 80,5 (d, 1C, ¹*J*_{CP} = 21,7 Hz), 73,8, 73,7 (s, 1C chacun), 72,2, 71,3 (s, 2C chacun), 52,6 (s, 1C), 48,9 (s, 1C), 43,3 (d, 1 C, T^{S}*J*_{CP} = 3,7 Hz), 33,9 (s, 1C), 28,1 (d, 2C, ^{TS}*J*_{CP} = 3,7 Hz), 24,2 (m, 1C), 23,1 (d, 2C, ¹*J*_{CP} = 16 Hz), 20, 20,2 (d, 2C chacun, ²*J*_{CP} = 14,3 Hz). C₅₅H₆₃P₃FeN (MW 885,85, masse exacte 885,34): *m*/*z* 886,349 (M+H)⁺; simulé 886,351 (σ = 0,574).

### Exemple 17 : Immobilisation du composé de formule 10 avec E = (CH₂)₃ et GF = 4-vinylbenzylamine sur support organique polymère.

Dans un Erlenmeyer de 500 ml, 4,5 g de gomme arabique et 2,8 g de chlorure de sodium et 112 ml d'eau distillée sont portés à ébullition. La solution est filtrée sur Célite® et 45 ml sont introduit dans un réacteur à rodage plan de 100 ml. Le styrène (3,2 ml, 27,7 mmol), le 1,1',2-tris(diphénylphosphino)-2'-diisopropylphosphino-4-[2-méthyl-5-(4-vinylbenzylamino)pent-2-yl]ferrocène (0,4 g, 0,45 mmol) et le divinylbenzène (0,41 g, 3,15 mmol) sont solubilisés dans 3 ml de chlorobenzène. La solution de monomère est injectée dans la solution de surfactant sous agitation mécanique. Le mélange est chauffé à 85 °C et du peroxyde de benzoyle (400 mg, 1,65 mmol) est ajouté. Après 20 h à 85 °C la solution est filtrée, et le polymère est lavé avec 2×30 ml d'eau chaude. Le polymère est ensuite extrait au Soxhlet dans le méthanol pendant 24 h puis séché sous vide pour donner 1,6 g de ligand polymérique insoluble dans les solvants organiques. La résine obtenue est insoluble dans tous les solvants organiques usuels testés (THF, CHCl₃, CH₂Cl₂, toluène, Et₂O, acétate d'éthyle, hexane, méthanol, DMF, DMSO, NMP, etc.). L'analyse RMN CP-MAS ³¹P obtenu à une vitesse de rotation de 14 Hz révèle la présence d'un signal centré sur -30 ppm. Ce déplacement est concordant avec le déplacement observé pour le ligand correspondant en solution. L'analyse élémentaire de ce polymère a révélé une teneur en phosphore de 1,0% ce qui correspond à une charge en ligand de 0,0595 mmol/g.

### Exemple 18 : réaction de couplage d'arylation du phényle acétylène à la bromoacétophénone par le composé de formule (10) supporté sur résine polystyrène.

Cette réaction de couplage carbone-carbone a été réalisée dans les conditions de basses charges métalliques < 1.0 mol% suivantes : un mélange de phényle acétylène (299 mg, 2.93 mmol, d = 0.93), de triéthyle amine (0,301 g, 2,93 mmol, d = 0.726), de bromoacétophénone (0,58 g, 2,95 mmol), du composé-ligand supporté sur silice issu du composé de formule **(10)** (0,24 g de résine polymère à 0,0595 mmol/g de ligand triphosphine, soit 0,0143 mmol) et du précurseur de palladium [Pd(C₃H₅)Cl]₂ (2,46 mg, 0,00715 mmol) est placé dans un tube de Schlenk sous argon. 2 mL de DMF distillé et dégazé par barbotage à l'azote est additionné et le mélange est maintenu pour 20 h à 120 °C. Le mélange refroidi est filtré pour récupérer le composé-ligand et le métal (moins de 1 ppb de Pd analysé par ICP restant en solution). Le filtrat est évaporé à sec sous vide, et le résidu solubilisé dans 10 mL d'éther éthylique avec 10 mL d'eau pour extraction. Les phases organiques sont séchées sur sulfate de magnésium. L'analyse par GC préalable indique une conversion totale de la bromoacétophénone en produit de couplage, pour 95% de rendement après évaporation du solvant et traitement chromatographique. Le catalyseur isolé par filtration est réengagé en réaction selon la même procédure. L'analyse GC montre a nouveau une conversion totale de la bromoacétophénone en produit de couplage, obtenu avec 95% de rendement après évaporation du solvant et traitement chromatographique. Le catalyseur isolé peut ainsi être réengagé plusieurs fois (six fois) sans baisse d'activité notable, la limitation résidant dans les pertes lors de la filtration.

### Exemple 19 : Synthèse générique des composés de formule (11) à (16)

A une suspension de FeCl₂ (1 equiv.) dans du THF (45 ml/g) est ajoutée à -80 °C une solution de 1,2-bis(diphénylphosphino)-4-*tert*-butylcyclopentadiényle lithium (1 equiv.) dans du THF (15 ml/g). La solution, maintenue sous agitation 1 h après retour à température ambiante, devient rouge foncé. Une solution du cyclopentadienyl lithium approprié (1 equiv.) dans du THF (30 ml/g) est ajoutée à -80 °C. Après retour à température ambiante, la solution rouge foncé est portée au reflux pour 4 h. La solution est filtrée sur silice, concentrée sous pression réduite et purifiée par chromatographie sur gel de silice pour donner le ligand férrocènique correspondant. Les déplacement chimiques et les caractérisations correspondent aux données des composés analogues non-hétérogénéisables tels que décrits par Broussier et al. dans J. Organomet. Chem. 2000, 598, 365-373.

### Exemple 20 : Synthèse générique des composés de formule (17) à (18)

A une suspension de FeCl₂ (1 equiv.) dans du THF (45 ml/g) est ajoutée à -80 °C une solution de 1-(diphénylphosphino)-4-*tert*-butylcyclopentadiényle lithium (1 equiv.) dans du THF (15 ml/g). La solution, maintenue sous agitation 1 h après retour à température ambiante, devient rouge foncé. Une solution du cyclopentadienyl lithium approprié (1 equiv.) dans du THF (30 ml/g) est ajoutée à -80 °C. Après retour à température ambiante, la solution rouge foncé est portée au reflux pour 4 h. La solution est filtrée sur silice, concentrée sous pression réduite et purifiée par chromatographie sur gel de silice pour donner le ligand férrocènique correspondant. Les déplacement chimiques et caractérisations correspondent aux données des composés analogues non-hétérogénéisables tels que décrits par Broussier et al. dans J. Organomet. Chem. 2000, 598, 365-373.

En conclusion, dans le contexte de la chimie durable, de l'économie des ressources et en particulier des plus rares, comme les métaux précieux, des systèmes catalytiques performants travaillant avec des quantités de catalyseur de 0,1 à 0,0001 mol% par rapport au substrat ont été développés depuis plusieurs années avec des ligands polyphosphines ferrocéniques, en particulier de formules (a) à (1).

Avec l'invention, l'utilisation de ces systèmes catalytiques de formules (a) à (1), mais supportés et récupérables, permet d'améliorer encore davantage les aspects de la « chimie verte » par la récupération des métaux et la facilité de séparation des produits formés et du catalyseur, même utilisés en quantité plus élevée mais en dessous de 1 mol% de catalyseur.

L'immobilisation de ces ligands permet d'augmenter la densité locale d'atomes coordinants sur un support solide, par l'implantation contrôlée de 3 à 4 atomes de phosphore dans un rayon de 3 à 5 angströms. Ces ligands immobilisés du type résine polyphosphoré, et en particulier pour des tétraphosphines (4 atomes de phosphore contraints à une grande proximité) n'ont jamais, à la connaissance des inventeurs, été synthétisés auparavant. Leur utilité en catalyse a été démontrée expérimentalement dans le couplage croisé.

De plus, à partir des ces nouveaux ligands phosphinique hétérogénéisables sont accessibles à la fois des résines purement hétérogènes (insolubles) et des résines polymériques solubles sélectivement selon les solvants choisis.

Le concept d'immobilisation de ces supports a été validé au travers d'une synthèse originale des ligands cibles permettant d'une part l'introduction d'un groupe fonctionnel servant de point d'ancrage à leur fixation sur support solide, et d'autre part la préservation de la conformation particulière de ces ligands, comme attesté par la diffraction X et par les systèmes de spin particuliers observés en RMN ³¹P.

Cette conformation, due à la présence de groupes encombrants attachés directement aux cyclopentadiènes, a pu être préservée en introduisant des groupes *gem*-diméthyles mimant la présence d'un groupe *tert*-butyle. La RMN ³¹P des divers ligands hétérogénéisables formés, ainsi que leur étude en diffraction X pour certains, a permis de confirmer le succès de cette stratégie.

## Revendications

1. Composés de formule (I) suivante : 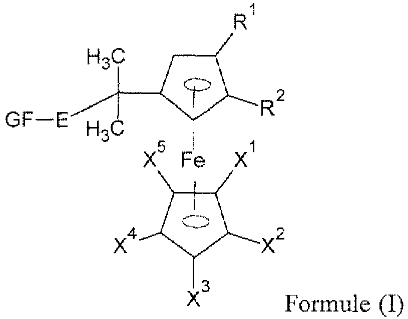 dans laquelle :
- E est une chaîne alkyle en C₁ à C₅, linéaire,
- GF est un groupe réactif choisi parmi les groupes vinyle et alkoxysilane dont la chaîne alkoxy est en C₁ à C₅, , linéaire ou ramifiée, saturée ou insaturée, contenant optionnellement au moins un hétéroatome, de préférence N et/ou O ;
- R¹ est un groupe phosphine P(R⁴)₂,
- R² est H ou un groupe phosphine P(R⁶)₂,
- X¹, X², X³ et X⁴ sont identiques ou différents et sont choisis, indépendamment les uns des autres, parmi un atome d'hydrogène, un groupe P(R⁸)₂, un groupe -C(CH₃)₂-P(R³)₂ ou un groupe amino R⁷N(R⁵)₂,
- X₅ est soit un groupe GF-E-C(CH₃)₂- dans lequel GF et E sont identiques à GF et E définis précédemment, soit H, soit un groupe phosphines P(R¹⁰)₂ ou un groupe amino R¹¹N(R⁹)₂,
- R³, R⁴, R⁵, R⁶, R⁷, R⁸, R⁹, R¹⁰ et R¹¹ sont, indépendamment les uns des autres, choisis parmi un groupe alkyle en C₁ à C₃₀, de préférence en C₁ à C₁₀, plus préférablement en C₁ à C₅, linéaire ou ramifiée, saturée ou insaturée, contenant optionnellement au moins un hétéroatome, de préférence un atome de N et/ou O, ou un groupe aryle, ou aliphatique cyclique ou acyclique, en C₃ à C₇, contenant optionnellement un hétéroatome, de préférence un atome de N et/ou O et optionnellement substitué, de préférence par au moins un groupe méthyle, méthoxy, halogène (F, Cl, Br, I) de préférence F ou Cl, ou CF₃ ; de préférence ils sont choisis parmi un groupe -CH₂-, éthyle (Et), isopropyle (i-Pr), cyclohexyle (Cy), tertiobutyle (t-Bu), ou phényle (Ph), ou furyle (Fu), optionnellement substitués, de préférence par au moins un groupe méthyle, méthoxy, halogène (F, Cl, Br, I) de préférence F ou Cl, ou CF₃ ; plus préférablement R³, R⁴, R⁶, R⁸ et R¹⁰ sont un groupe phényle, R⁵ et R⁹ sont tous deux un groupe éthyle, R⁷, R¹¹ sont tous deux un groupe -CH₂-.

2. Composés selon la revendication 1, **caractérisés en ce que**
- dans la formule (I) X₅ est GF-E-C(CH₃)₂-,
- X¹ et X⁴ sont H et **en ce qu'**ils ont la formule (I-A) suivante : 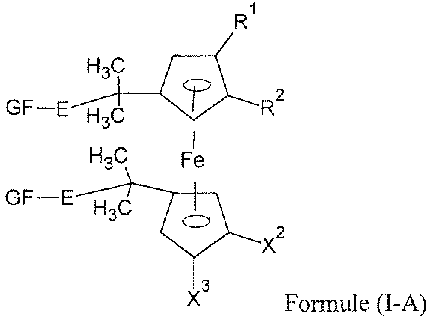 dans laquelle X² et X³ sont tels que définis en revendication 1.

3. Composés selon la revendication 1 ou 2, **caractérisés en ce que**, dans la formule (I) et dans la formule (I-A) X² = X³ = P(Ph)₂, X¹ et X⁴ sont 1H et R¹ = R² = P(Ph)₂ et **en ce qu'**ils ont les formules (1) à (8) suivantes, donnant un composé symétrique :

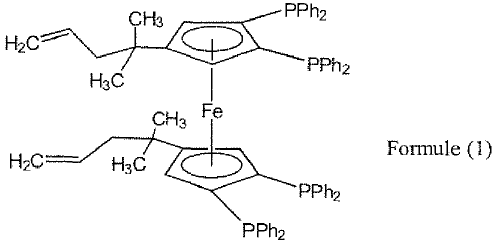

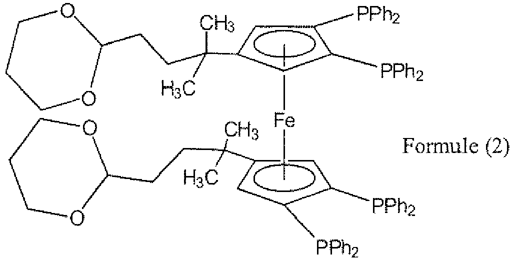

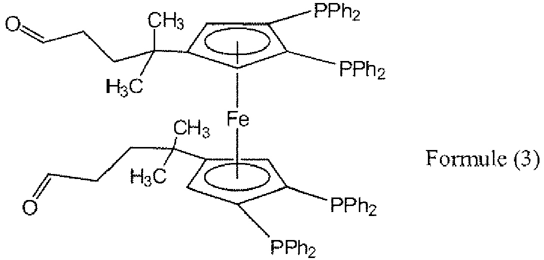

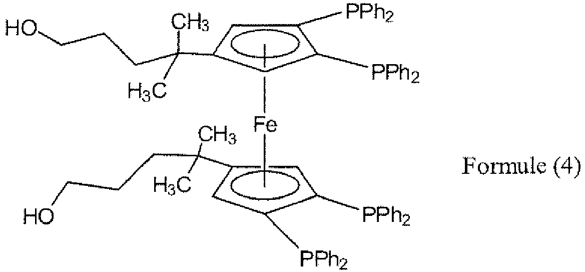

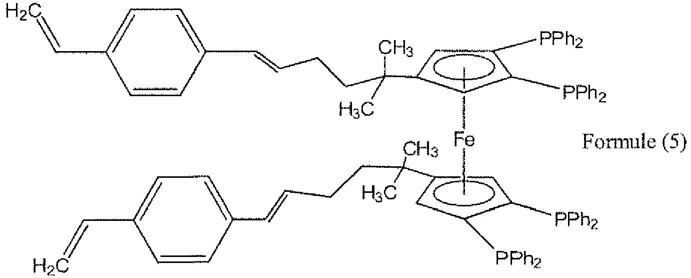

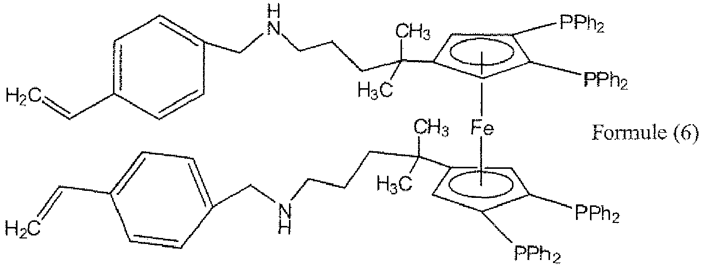

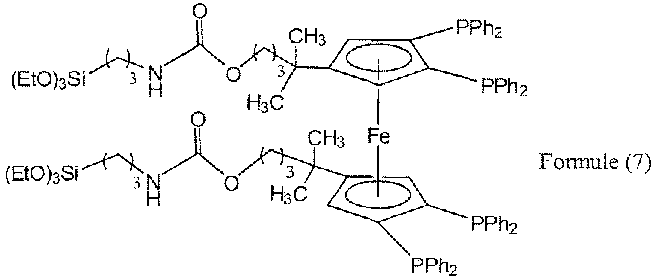

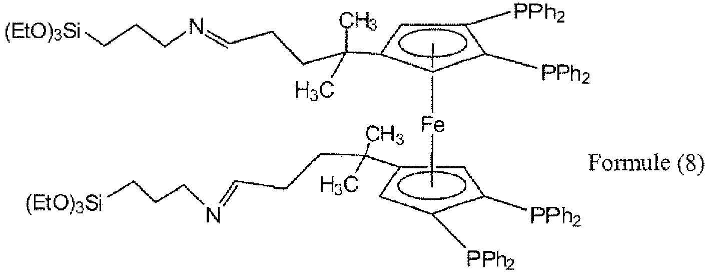

4. Composés selon la revendication 1, **caractérisés en ce que** dans la formule (I) :
- X⁵ est différent de GF-E-C(CH₃)₂, les rendant dissymétriques, et **en ce qu'**ils ont les formules (9) à (18) suivantes, dans lesquelles :
- R⁴ et R⁶ désignent, indépendamment les uns des autres, un groupe isopropyle, cyclohexyle, tertiobutyle ou phényle, ou furyle, optionnellement substitués, de préférence par au moins un groupe méthyle, méthoxy, un atome d'halogène tel que F, Cl, Br, I, de préférence F ou Cl, ou un groupe CF₃, de préférence R⁴ et R⁶ désignent un groupe phényle,
- R⁵ désigne :
- soit une chaîne alkyle en C₁ à C₃₀, de préférence en C₃ à C₁₀, plus préférablement en C₃ à C₅, linéaire ou ramifiée, saturée ou insaturée et pouvant contenir au moins un hétéroatome, de préférence N et/ou O,
- soit un groupe aryle, ou aliphatique cyclique ou acyclique en C₃ à C₇, contenant optionnellement un hétéroatome et optionnellement substitué, de préférence par au moins un groupe méthyle, méthoxy, un atome d'halogène tel que F, Cl, Br, I, de préférence F ou Cl, ou un groupe CF₃, de préférence R⁵ désigne un groupe éthyle, et **en ce qu'**ils ont la formule (9) à (18) suivantes :

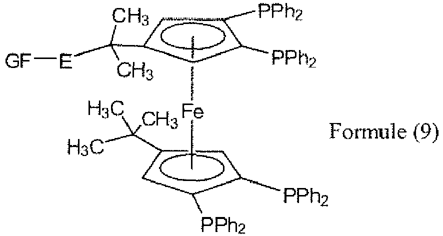

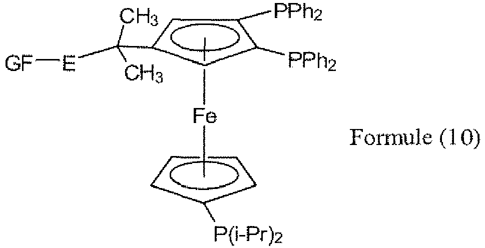

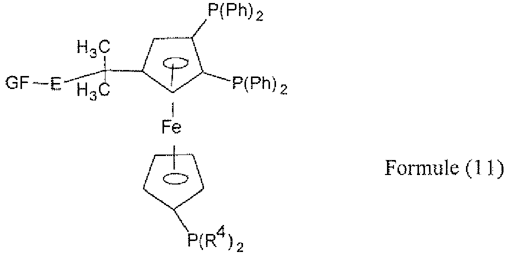

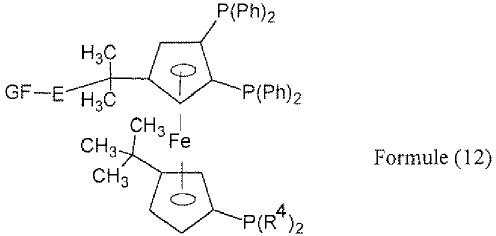

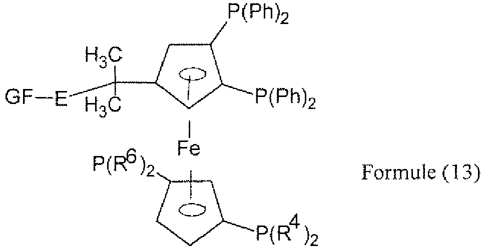

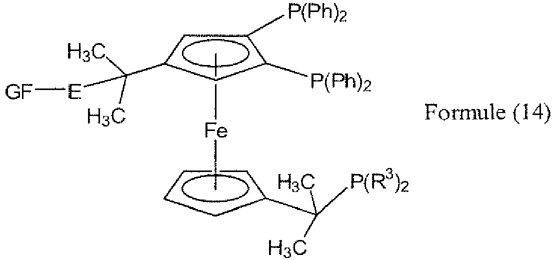

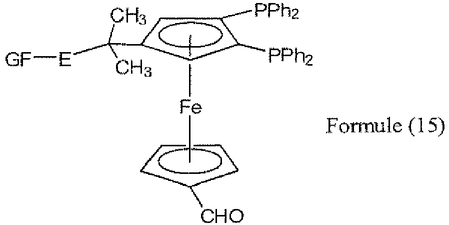

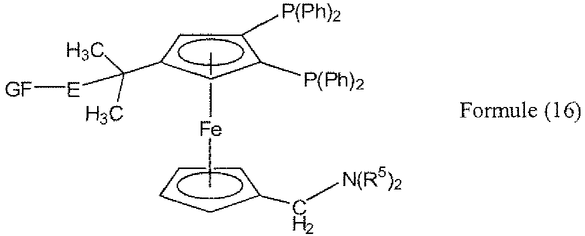

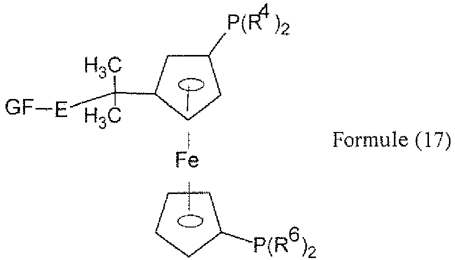

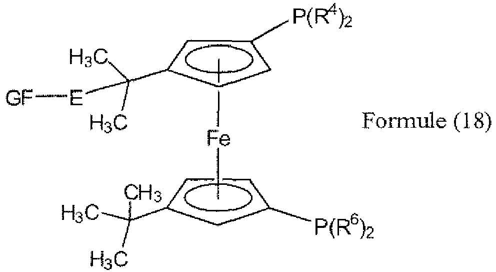

5. Complexes **caractérisés en ce qu'**ils comprennent un composé selon l'une quelconque des revendications précédentes complexé avec un métal choisi parmi les métaux de transition d, de préférence parmi le palladium, le cuivre, le nickel, le platine, le rhodium, l'iridium, le fer, le zirconium, le titane et le ruthénium et plus préférablement, le palladium.

6. Complexes supportés **caractérisés en ce qu'**ils comprennent un complexe selon la revendication 5 immobilisé sur un support par greffage ou polymérisation du groupe réactif GF d'un composé selon l'une quelconque des revendications 1 à 4 avec un groupe réactif d'un support.

7. Ligands supportés **caractérisés en ce qu'**ils comprennent un composé selon l'une quelconque des revendications 1 à 4 immobilisé par greffage ou polymérisation de son (ses) groupe(s) réactif(s) GF avec un (des) groupe(s) réactif(s) d'un support.

8. Procédé de synthèse des composés selon la revendication 2 ou 3, **caractérisé en ce qu'**il comprend les étapes suivantes :
a) préparation d'un sel de lithium cyclopentadiénylique portant les groupements E et GF souhaités ou une fonction réactive précurseur de ces groupements,
b) deux réactions successives de phosphination/lithiation du composé obtenu à l'étape a), et
c) formation du composé désiré par réaction du composé obtenu à l'étape b) avec un sel de fer.

9. Procédé de synthèse du composé de formule (1) **caractérisé en ce qu'**il comprend les étapes suivantes :
a) introduction du groupement fonctionnel GF qui est un groupe vinyle -CH=CH₂ et d'un espaceur E qui est -CH₂- par réaction du 6,6-diméthylfulvène avec un groupe méthyle l'organomagnésien CH₂=CH-CH₂-MgCl pour obtenir le 1-[2-(2-méthyl)pent-4-ényl]cyclopentadiène,
b) lithiation du produit obtenu à l'étape a) avec *n*-BuLi pour obtenir le 1-[2-[2-méthyl]pent-4-ényl]cyclopentadiényllithium,
c) deux phosphinations successives, entre lesquelles on réalise une étape de lithiation avec *n*-BuLi, du produit obtenu à l'étape b) avec ClP(Ph)₂,
d) traitement du composé obtenu à l'étape c) avec *n*-BuLi pour obtenir le 1,2-bis(diphénylphosphino)-4-[2-(2-méthyl)pent-4-ényl]cyclopentadiényllithium, et
e) synthèse du composé de formule (1) par mise en réaction de FeCl₂ avec le composé obtenu à l'étape d).

10. Procédé de synthèse du composé de formule (2) **caractérisé en ce qu'**il comprend les étapes suivantes :
a) réaction d'un organomagnésien de formule suivante : 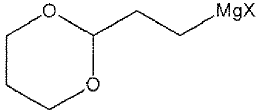 dans laquelle X est Br ou I
avec CH₃COCl, pour obtenir la 4-(1,3-dioxane-2-yl)butane-2-one de formule A suivante : 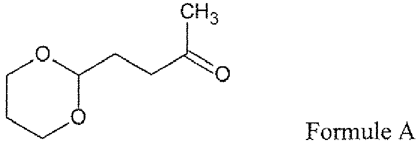
b) réaction du composé de formule A obtenu à l'étape a), avec de la pyrrolidine et du cyclopentadiényllithium (CpLi) pour obtenir le 2-[3-(cyclopenta-2,4-diénylidène)butyl]-1,3-dioxane de formule B suivante : 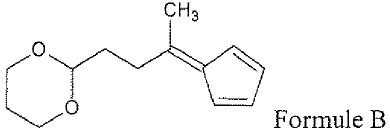
c) lithiation du composé de formule B avec du méthyllithium (MeLi) pour obtenir le composé de formule C suivante : 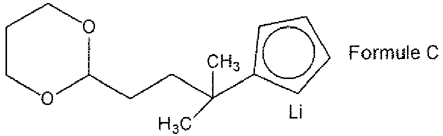
d) deux phosphinations successives du composé de formule C avec ClP(Ph)₂, avec passage intermédiaire au sel de lithium correspondant à la monophosphination par action de *n*-BuLi, suivies par un traitement avec *n*-BuLi pour obtenir le composé de formule D suivante : 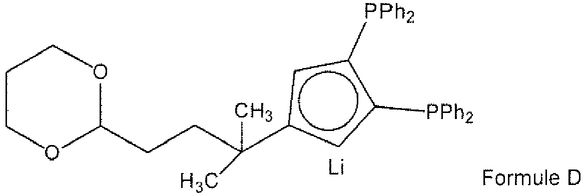
e) formation du composé de formule (2) suivante : 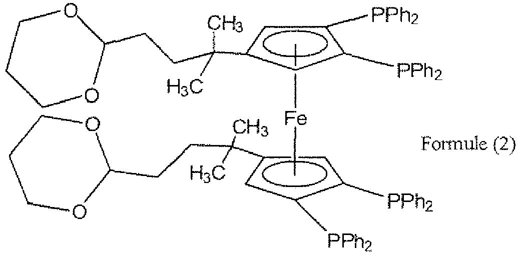 par réaction du composé de formule D avec un sel de fer.

11. Composés **caractérisés en ce qu'**ils ont l'une des formules B ou C ou D ou F suivantes :

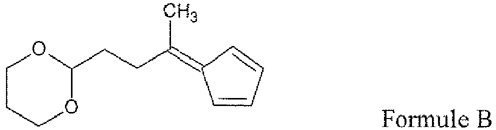

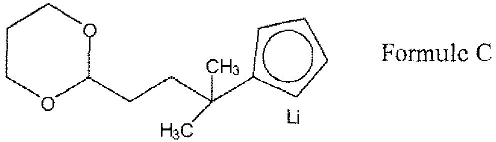

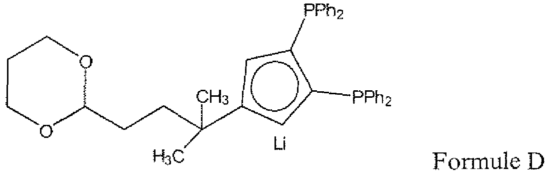

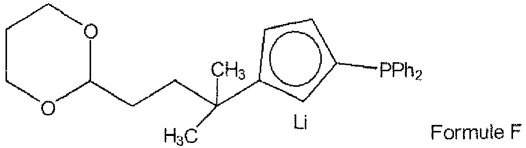

12. Procédé de synthèse du composé de formule (3) **caractérisé en ce qu'**il comprend la déprotection du composé de formule (2) dans des conditions acides.

13. Procédé de synthèse du composé de formule (3) supporté **caractérisé en ce qu'**il comprend la déprotection du composé de formule (2) dans des conditions acides et le greffage du composé de formule (3) ainsi obtenu sur un support comportant des groupes NH₂.

14. Procédé de synthèse du composé de formule (4) **caractérisé en ce qu'**il comprend la réduction du composé de formule (3).

15. Procédé de synthèse du composé de formule (4) supporté **caractérisé en ce qu'**il comprend le greffage du composé de formule (4) avec un support comprenant des groupes réactifs susceptibles de réagir avec un groupe alcool, de préférence des groupes isocyanates ou silanes.

16. Procédé de synthèse du composé de formule (5) **caractérisé en ce qu'**il comprend la réaction du composé de formule (3) avec un composé de formule (1-4) suivante :

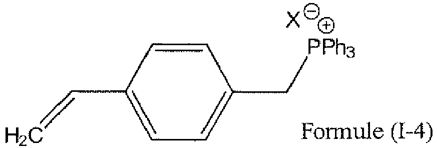

17. Procédé de synthèse du composé de formule (5) **caractérisé en ce qu'**il comprend la réaction du composé de formule (3) avec un composé de formule (I-5) suivante :

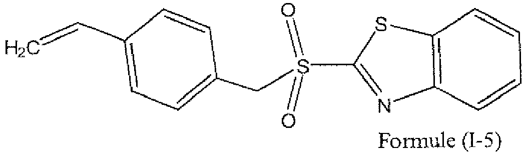

18. Procédé de synthèse du composé de formule (6) **caractérisé en ce qu'**il comprend la réaction du composé de formule (3) avec un composé *p*-vinylbenzylamine de formule (1-2) suivante :

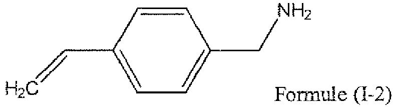

19. Procédé de synthèse du composé de formule (6) supporté **caractérisé en ce qu'**il comprend la polymérisation du composé de formule (6) avec le composé styrène de formule (I-3) suivante :

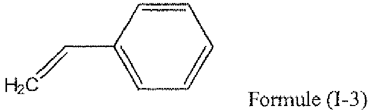

20. Procédé de synthèse du composé de formule (7) **caractérisé en ce qu'**il comprend la réaction du composé de formule (4) avec un composé alkoxysilane de formule (I-6) suivante :

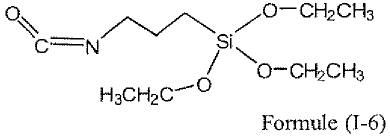

21. Procédé de synthèse du composé de formule (8) **caractérisé en ce qu'**il comprend la réaction du composé de formule (3) avec un composé alkoxysilane de formule (I-7) suivante :

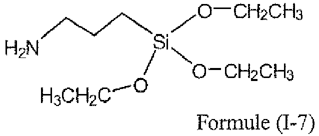

22. Procédé de synthèse d'un composé ayant l'une des formules (9) à (16), **caractérisé en ce qu'**il comprend les étapes suivante :
a) synthèse du composé de formule D suivante : 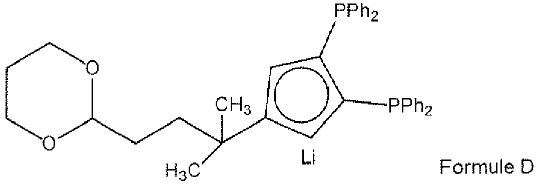
b) synthèse d'un sel d'un métal M, de préférence M est le lithium ou le sodium, du cyclopentadiényle ayant la formule G suivante : 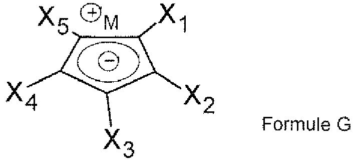 dans laquelle X¹, X², X³, X⁴ et X⁵ sont tels que définis en revendication 4,
c) réaction du composé obtenu à l'étape b) avec un sel de fer et le composé D.

23. Procédé de synthèse du composé de formule (9), **caractérisé en ce qu'**il comprend les étapes de réaction du procédé selon la revendication 22, dans lequel, dans le composé G, X¹ = X⁴ = H et X² = X³ = P(Ph)₂ et X⁵ = t-Bu et M = Li.

24. Procédé de synthèse du composé de formule (10), **caractérisé en ce qu'**il comprend les étapes de réaction du procédé de synthèse selon la revendication 22 dans lequel, dans le composé G, X¹ = X² = X⁴ = X⁵ = H et X³ = P(i-Pr)₂ et M = Li.

25. Procédé de synthèse d'un composé de formule (11), **caractérisé en ce qu'**il comprend les étapes de réaction du procédé de synthèse selon la revendication 22 dans lequel, dans le composé G, X¹ = X² = X⁴ = X⁵ = H et X³ = P(R⁴)₂ dans lequel R⁴ est choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₇, saturé ou insaturé et pouvant contenir au moins un hétéroatome, de préférence N et/ou O ; de préférence R⁴ est un groupe cyclohexyle, tertiobutyle, ou phényle ou furyle, optionnellement substitués, de préférence par au moins un groupe méthyle, méthoxy, un atome d'halogène tel que F, Cl, Br, I, de préférence F ou Cl, ou un groupe CF₃.

26. Procédé de synthèse d'un composé de formule (12), **caractérisé en ce qu'**il comprend les étapes de réaction du procédé de synthèse selon la revendication 22 dans lequel, dans le composé G, M = Li, X¹ = X² = X⁴ = H, X³ = P(R⁴)₂ et X⁵ = t-Bu dans lequel R⁴ est choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O ; de préférence R⁴ est un groupe isopropyle, cyclohexyle, tertiobutyle, ou phényle ou furyle, optionnellement substitués, de préférence par au moins un groupe méthyle, ou méthoxy, ou un atome d'halogène tel que F, Cl, Br, I, de préférence F ou Cl, ou un groupe CF₃.

27. Procédé de synthèse d'un composé de formule (13), **caractérisé en ce qu'**il comprend les étapes de réaction du procédé de synthèse selon la revendication 22 dans lequel, dans le composé G, M = Li, X¹ = X² = X⁴ = H, X³ = P(R⁴)₂ et X⁵ = P(R⁶)₂, dans lesquels R⁴ et R⁶ sont, indépendamment l'un de l'autre, choisis parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O ; de préférence R⁴ et R⁶ sont, indépendamment l'un de l'autre, un groupe isopropyle, cyclohexyle, tertiobutyle, ou phényle ou furyle, optionnellement substitués, de préférence par au moins un groupe méthyle, ou méthoxy, ou un atome d'halogène tel que F, Cl, Br, I, de préférence F ou Cl, ou un groupe CF₃.

28. Procédé de synthèse d'un composé de formule (14), **caractérisé en ce qu'**il comprend les étapes de réaction du procédé de synthèse selon la revendication 22 dans lequel, dans le composé G, M = Li, X¹ = X² = X⁴ = X⁵ = H, X² = C(CH₃)₂-P(R³)₂ et R³ est choisi parmi un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O ; de préférence R³ est un groupe isopropyle, cyclohexyle, tertiobutyle, ou phényle ou furyle, optionnellement substitués, de préférence par au moins un groupe méthyle, ou méthoxy, ou un atome d'halogène tel que F, Cl, Br, I, de préférence F ou Cl, ou un groupe CF₃.

29. Procédé de synthèse du composé de formule (15), **caractérisé en ce qu'**il comprend les étapes de réaction du procédé de synthèse selon la revendication 22 dans lequel, dans le composé G, M = Na, X¹ = X² = X⁴ = X⁵ = H, et X³ = CHO.

30. Procédé de synthèse d'un composé de formule (16), **caractérisé en ce qu'**il comprend la réaction du composé de formule (15) avec une amine secondaire en présence de NaBH(OAc)₃, l'amine secondaire ayant la formule HN(R⁵)₂ dans laquelle R⁵ est un groupe aryle, ou aliphatique cyclique ou acyclique en C₃ à C₇, ou un groupe alkyle linéaire ou ramifié en C₁ à C₃₀, de préférence en C₃ à C₁₀, plus préférablement en C₃ à C₅, saturé ou insaturé et pouvant contenir au moins un hétéroatome, de préférence N et/ou O ; de préférence R⁵ est un groupe éthyle.

31. Procédé de synthèse des composés de formules (17) et (18), **caractérisé en ce qu'**ils comprennent les étapes suivantes :
a) synthèse du composé de formule F suivante : 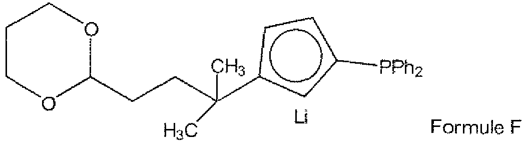
b) synthèse d'un sel d'un métal M, de préférence lithium ou sodium, de cyclopentadiényle ayant la formule G suivante : 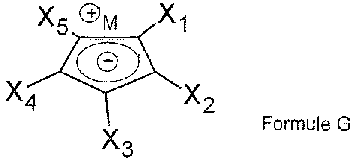 dans laquelle X¹, X², X³, X⁴ et X⁵ sont tels que définis précédemment dans les composés de formule (9) à (14), pour les composés de formules (17) ou (18) de type tri ou diphosphines dissymétriques,
c) réaction du composé obtenu à l'étape b) avec un sel de fer et le composé F.

32. Procédé de synthèse de complexes métalliques des composés de formules (1) à (18), **caractérisé en ce qu'**il comprend la complexation de chacun des composés de formules (1) à (18) avec un métal choisi parmi les métaux de transition d, de préférence, le palladium, le cuivre, le nickel, le platine, l'iridium, le rhodium, le fer, le zirconium, le titane et le ruthénium, plus préférablement le palladium.

33. Procédé de synthèse de complexes métalliques supportés issus des composés de formules (1) à (18), **caractérisé en ce qu'**il comprend le greffage sur un support, ou la copolymérisation, de chacun des complexes supportés selon la revendication 5 ou obtenus par le procédé de la revendication 32.

34. Utilisation d'au moins un composé selon l'une quelconque des revendications 1 à 4 pour la fabrication d'un catalyseur supporté.

35. Utilisation d'au moins un complexe selon la revendication 5 pour la fabrication d'un catalyseur supporté.

36. Procédé de couplage catalytique de composés nucléophiles et électrophiles, de préférence de couplage carbone-carbone, carbone-azote et carbone-oxygène, **caractérisé en ce qu'**on utilise un catalyseur ou complexe supporté selon la revendication 6 ou obtenu par le procédé selon la revendication 33 à 35.

## Patentansprüche

1. Verbindungen mit der folgenden Formel (I): 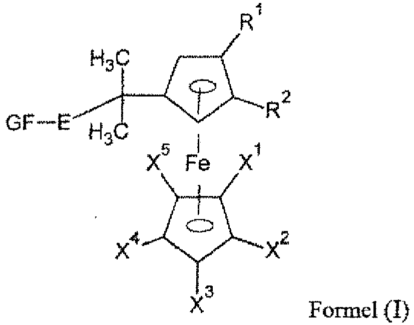 wobei:
- E eine lineare Alkylkette mit C₁ bis C₅ ist,
- GF eine reaktive Gruppe ist, ausgewählt aus den Vinyl- und Alkoxysilangruppen, deren Alkoxykette mit C₁ bis C₅, linear oder verzweigt, gesättigt oder nicht gesättigt ist, enthaltend optional mindestens ein Heteroatom, vorzugsweise N und/oder O;
- R¹ eine Phosphingruppe P(R⁴)₂ ist,
- R² H oder eine Phosphingruppe P(R⁶)₂ ist,
- X¹, X², X³ und X⁴ identisch oder verschieden sind und unabhängig voneinander ausgewählt sind aus einem Wasserstoffatom, einer P(R⁸)₂-, einer - C(CH₃)₂-P(R³)₂- oder einer R⁷N(R⁵)₂-Aminogruppe,
- X₅ entweder eine GF-E-C(CH₃)₂-Gruppe ist, in der GF und E identisch mit GF und E, wie oben definiert, sind, oder H oder eine P(R¹⁰)₂-Phosphingruppe oder eine R¹¹N(R⁹)₂-Aminogruppe,
- R³, R⁴, R⁵, R⁶, R⁷, R⁸, R⁹, R¹⁰ und R¹¹ unabhängig voneinander ausgewählt sind aus einer Alkylgruppe mit C₁ bis C₃₀, vorzugsweise mit C₁ bis C₁₀, insbesondere mit C₁ bis C₅, linear, verzweigt, gesättigt oder nicht gesättigt, enthaltend optional mindestens ein Heteroatom, vorzugsweise ein Atom von N und/oder O; und eine Aryl- oder aliphatische Gruppe, zyklisch oder azyklisch, mit C₃ bis C₇, enthaltend optional mindestens ein Heteroatom, vorzugsweise ein Atom von N und/oder O und optional substituiert, vorzugsweise durch mindestens eine Methyl-, Methoxy-, Halogengruppe (F, Cl, Br, I), vorzugsweise F oder Cl oder CF₃; vorzugsweise sind sie ausgewählt aus einer -CH₂₋, Ethyl- (Et), Isopropyl (i-Pr)-, Cyclohexyl (Cy)-, Tertiobutyl (t-Bu)-, oder Phenyl (Ph)- oder Furyl (Fu)-Gruppe, optional substituiert, vorzugsweise durch mindestens eine Methyl-, Methoxy-, Halogengruppe (F, Cl, Br, I), vorzugsweise F oder Cl oder CF₃; insbesondere sind R³, R⁴, R⁶, R⁸ und R¹⁰ eine Phenylgruppe, R⁵ und R⁹ sind beide eine Ethylgruppe, R⁷, R¹¹ sind beide eine -CH₂-Gruppe.

2. Verbindungen nach Anspruch 1, **dadurch gekennzeichnet, dass**
- in der Formel (I) X₅, GF-E-C(CH₃)₂- ist,
- X¹ und X⁴, H sind, und dadurch, dass sie die folgende Formel (I-A) aufweisen: 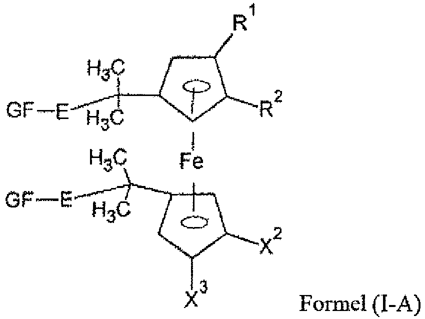 wobei X² und X³ wie in Anspruch 1 definiert sind.

3. Verbindungen nach Anspruch 1 oder 2, **dadurch gekennzeichnet, dass** in der Formel (I) und in der Formel (1-A), X² = X³ = P(Ph)₂, X¹ und X⁴, H und R¹ = R² = P(Ph)₂ sind, und dadurch, dass die folgenden Formeln (1) bis (8) eine symmetrische Verbindung ergeben:

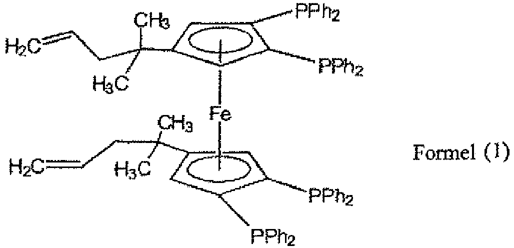

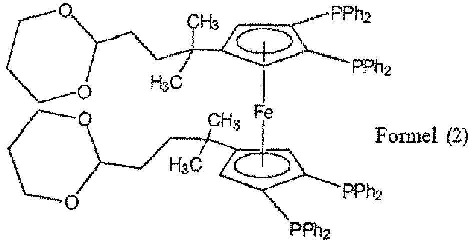

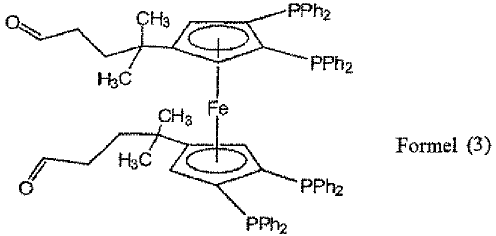

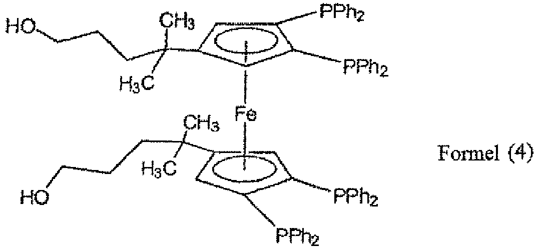

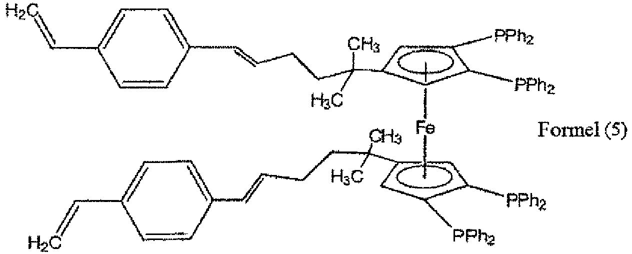

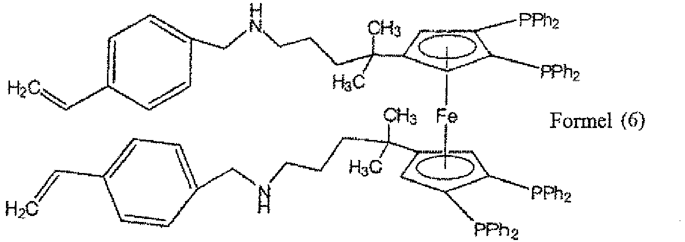

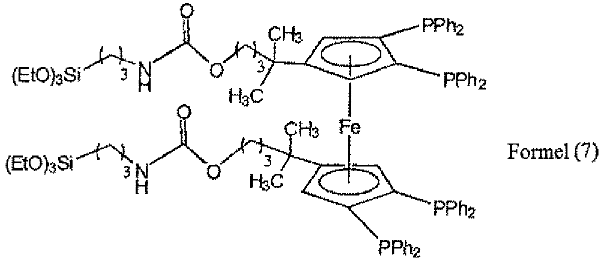

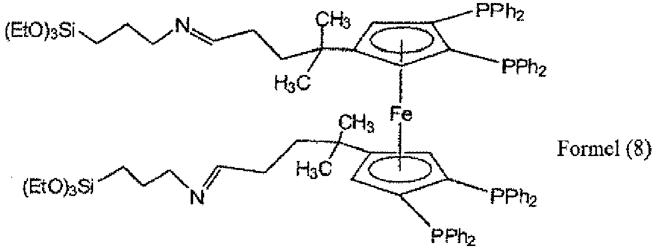

4. Verbindungen nach Anspruch 1, **dadurch gekennzeichnet, dass** in der Formel (I):
- X⁵ verschieden von GF-E-C(CH₃)₂ ist, wodurch sie unsymmetrisch werden, und dadurch, dass sie die folgenden Formeln (9) bis (18) aufweisen, in denen:
- R⁴ und R⁶ unabhängig voneinander eine Isopropyl-, Cyclohexyl-, Tertiobutyl oder Phenyl- oder Furylgruppe bezeichnen, optional substituiert, vorzugsweise durch mindestens eine Methyl-, Methoxygruppe, ein Halogenatom wie z. B. F, Cl, Br, I, vorzugsweise F oder Cl, oder eine CF₃-Gruppe, vorzugsweise R⁴ und R⁶ eine Phenylgruppe bezeichnen,
- R⁵ Folgendes bezeichnet:
- entweder eine Alkylkette mit C₁ bis C₃₀, vorzugsweise mit C₃ bis C₁₀, insbesondere mit C₃ bis C₅, linear, verzweigt, gesättigt oder nicht gesättigt, und die optional mindestens ein Heteroatom, vorzugsweise N und/oder O enthalten kann;
- oder eine Aryl- oder aliphatische Gruppe, zyklisch oder azyklisch, mit C₃ bis C₇, enthaltend optional ein Heteroatom, und optional substituiert, vorzugsweise durch mindestens eine Methyl-, Methoxygruppe, ein Halogenatom wie z. B. F, Cl, Br, I, vorzugsweise F oder Cl, oder eine CF₃-Gruppe, vorzugsweise R⁵, eine Ethylgruppe bezeichnet, und dadurch, dass sie die folgenden Formeln (9) bis (18) aufweisen:

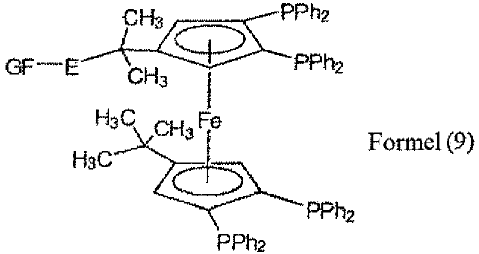

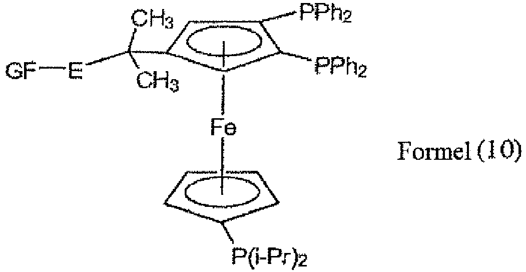

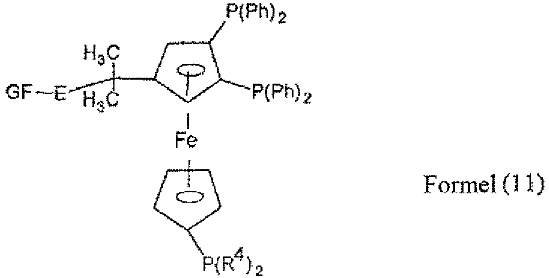

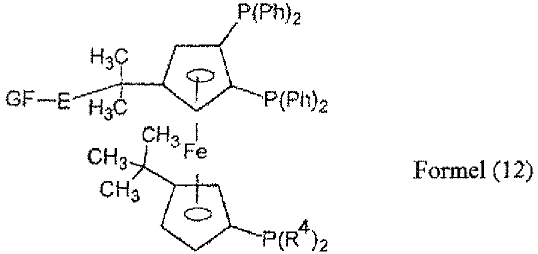

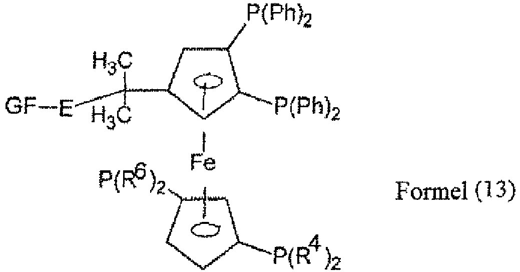

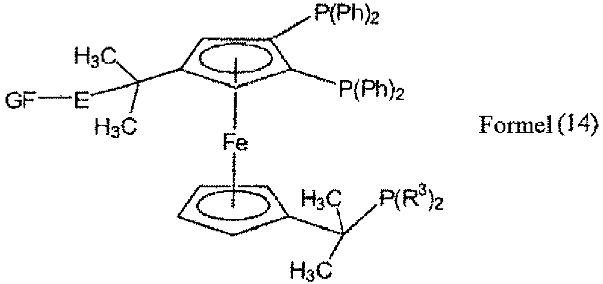

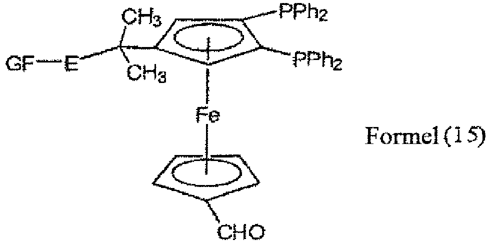

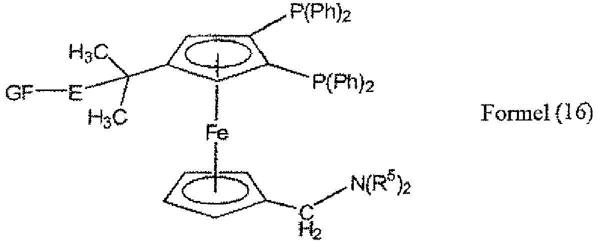

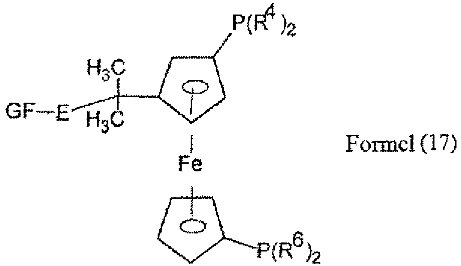

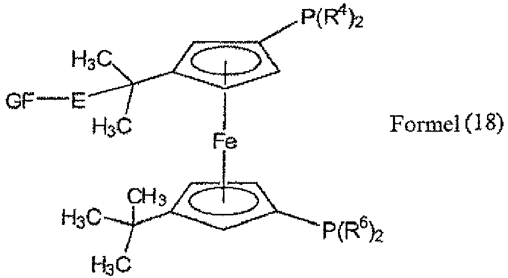

5. Komplexe, **dadurch gekennzeichnet, dass** sie eine Verbindung nach einem beliebigen der vorhergehenden Ansprüche umfassen, komplexiert mit einem Metall, ausgewählt aus den Übergangsmetallen d, vorzugsweise aus Palladium, Kupfer, Nickel, Platin, Rhodium, Iridium, Eisen, Zirconium, Titan und Ruthenium und insbesondere Palladium.

6. Trägerkomplexe, **dadurch gekennzeichnet, dass** sie einen Komplex nach Anspruch 5 umfassen, immobilisiert auf einem Träger durch Implantieren oder Polymerisieren der Reaktionsgruppe GF einer Verbindung nach einem beliebigen der Ansprüche 1 bis 4 mit einer Reaktionsgruppe eines Trägers.

7. Trägerliganden, **dadurch gekennzeichnet, dass** sie eine Verbindung nach einem beliebigen der Ansprüche 1 bis 4 umfassen, immobilisiert durch Implantieren oder Polymerisieren ihrer Reaktionsgruppe(n) GF mit einer Reaktionsgruppe/Reaktionsgruppen eines Trägers.

8. Verfahren zur Synthese der Verbindungen nach Anspruch 2 oder 3, **dadurch gekennzeichnet, dass** es die folgenden Schritte umfasst;
a) Herstellen eines Cyclopentadienyl-Lithiumsalzes, das die gewünschten E- und GF-Gruppierungen oder eine Vorläufer-Reaktionsfunktion dieser Gruppierungen trägt.
b) zwei aufeinander folgende Phosphinierungs-/Lithiierungsreaktionen der Verbindung, erhalten in Schritt a), und
c) Bilden der gewünschten Verbindung durch Reaktion der Verbindung, erhalten in Schritt b) mit einem Eisensalz.

9. Verfahren zur Synthese der Verbindung mit der Formel (1), **dadurch gekennzeichnet, dass** es die folgenden Schritte umfasst:
a) Einführen der funktionellen GF-Gruppierung, wobei es sich um eine -CH=CH₂₋ Vinylgruppe handelt, und eines Spacers E, wobei es sich um -CH₂- handelt, durch Reaktion des 6,6-Dimethylfulvens mit einer Methylgruppe des Organomagnesiums CH₂=CH-CH₂-MgCl, um das 1-[2-(2-Methyl)pent-4-enyl]cyclopentadien zu erhalten,
b) Lithiieren des Produkts, erhalten in Schritt a) mit *n*-BuLi, um das 1-[2-[2-Methyl]pent-4-enyl]cyclopentadienyllithium zu erhalten,
c) zwei aufeinander folgende Phosphinierungen, zwischen denen ein Schritt des Lithiierens mit *n*-BuLi durchgeführt wird, des Produkts, erhalten in Schritt b) mit ClP(Ph)₂,
d) Behandeln der Verbindung, erhalten in Schritt c), mit *n*-BuLi, um 1,2-Bis(diphenylphosphino)-4-[2-(2-methyl)pent-4-enyl]cyclopentadienyllithium zu erhalten, und
e) Synthese der Verbindung mit der Formel (1) durch Reaktion von FeCl₂ mit der Verbindung, erhalten in Schritt d).

10. Verfahren zur Synthese der Verbindung mit der Formel (2), **dadurch gekennzeichnet, dass** es die folgenden Schritte umfasst:
a) Reagieren eines Organomagnesiums mit der folgenden Formel: 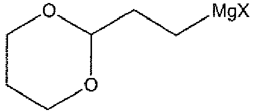 wobei X, Br oder I ist
mit CH₃COCl, um 4-(1,3-Dioxan-2-yl)butan-2-on mit der folgenden Formel A zu erhalten: 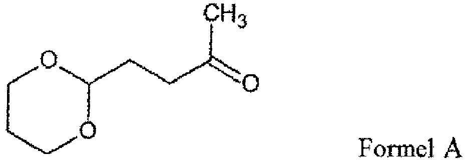
b) Reagieren der Verbindung mit der Formel A, erhalten in Schritt a), mit Pyrrolidin und Cyclopentadienyllithium (CpLi), um das 2-[3-(Cyclopenta-2,4-dienyliden)butyl]-1,3-dioxan mit der folgenden Formel B zu erhalten: 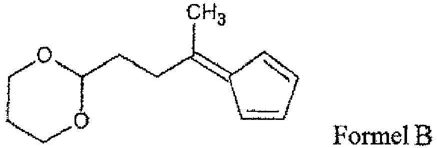
c) Lithiieren der Verbindung mit der Formel B mit Methyllithium (MeLi), um die Verbindung mit folgenden Formel C zu erhalten: 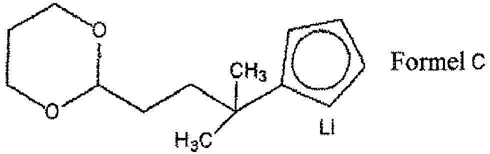
d) zwei aufeinander folgende Phosphinierungen der Verbindung mit der Formel C mit ClP(Ph)₂, mit Zwischendurchgang des Lithiumsalzes, das der Monophosphinierung entspricht, durch Wirkung von *n*-BuLi, gefolgt von einer Behandlung mit *n*-BuLi, um die Verbindung mit der folgenden Formel D zu erhalten: 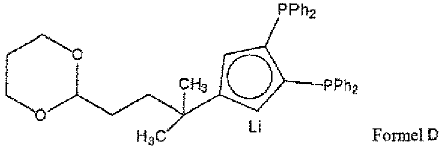
e) Bilden der Verbindung mit der folgenden Formel (2): 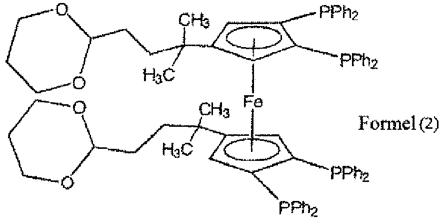 durch Reagieren der Verbindung mit der Formel D mit einem Eisensalz.

11. Verbindungen, **dadurch gekennzeichnet, dass** sie eine der folgenden Formeln B oder C oder D oder F aufweisen:

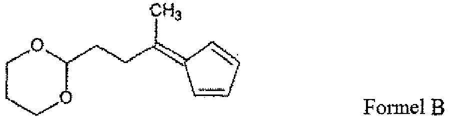

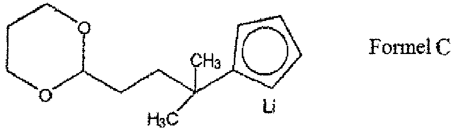

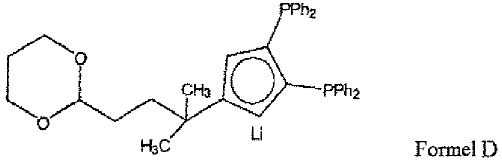

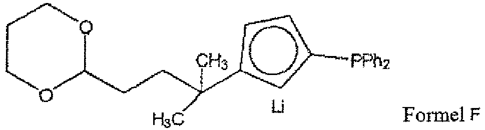

12. Verfahren zur Synthese der Verbindung mit der Formel (3), **dadurch gekennzeichnet, dass** es das Entschützen der Verbindung mit der Formel (2) unter sauren Bedingungen umfasst.

13. Verfahren zur Synthese der Verbindung mit der Trägerformel (3), **dadurch gekennzeichnet, dass** es das Entschützen der Verbindung mit der Formel (2) unter sauren Bedingungen und das Implantieren der so erhaltenen Verbindung mit der Formel (3) auf einem Träger, umfassend NH₂-Gruppen, umfasst.

14. Verfahren zur Synthese der Verbindung mit der Formel (4), **dadurch gekennzeichnet, dass** es die Reduktion der Verbindung mit der Formel (3) umfasst.

15. Verfahren zur Synthese der Trägerverbindung mit der Formel (4), **dadurch gekennzeichnet, dass** es das Implantieren der Verbindung mit der Formel (4) mit einem Träger umfasst, umfassend Reaktionsgruppen, die mit einer Alkoholgruppe reagieren können, vorzugsweise Isocyanat- oder Silangruppen.

16. Verfahren zur Synthese der Verbindung mit der Formel (5), **dadurch gekennzeichnet, dass** es die Reaktion der Verbindung mit der Formel (3) mit einer Verbindung mit der folgenden Formel (I-4) umfasst:

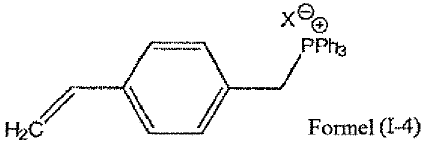

17. Verfahren zur Synthese der Verbindung mit der Formel (5), **dadurch gekennzeichnet, dass** es die Reaktion der Verbindung mit der Formel (3) mit einer Verbindung mit der folgenden Formel (1-5) umfasst:

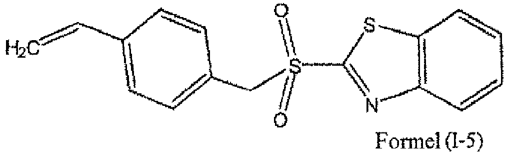

18. Verfahren zur Synthese der Verbindung mit der Formel (6), **dadurch gekennzeichnet, dass** es die Reaktion der Verbindung mit der Formel (3) mit einer *p*-Vinylbenzylamin-Verbindung mit der folgenden Formel (I-2) umfasst:

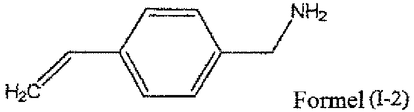

19. Verfahren zur Synthese der Trägerverbindung mit der Formel (6), **dadurch gekennzeichnet, dass** es die Polymerisierung der Verbindung mit der Formel (6) mit der Styrenverbindung mit der folgenden Formel (I-3) umfasst:

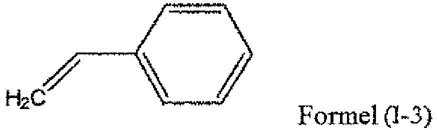

20. Verfahren zur Synthese der Verbindung mit der Formel (7), **dadurch gekennzeichnet, dass** es die Reaktion der Verbindung mit der Formel (4) mit einer Alkoxysilan-Verbindung mit der folgenden Formel (I-6) umfasst:

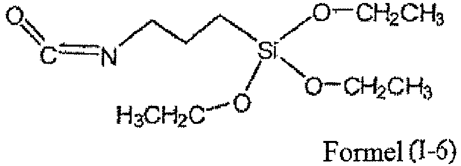

21. Verfahren zur Synthese der Verbindung mit der Formel (8), **dadurch gekennzeichnet, dass** es die Reaktion der Verbindung mit der Formel (3) mit einer Alkoxysilan-Verbindung mit der folgenden Formel (I-7) umfasst:

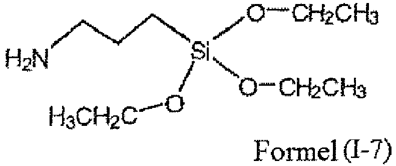

22. Verfahren zur Synthese einer Verbindung mit einer der Formeln (9) bis (16), **dadurch gekennzeichnet, dass** es die folgenden Schritte umfasst:
a) Synthese der Verbindung mit der folgenden Formel D: 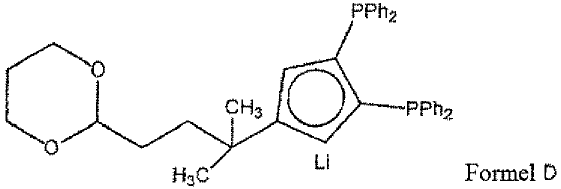
b) Synthese eines Salzes eines Metalls M, vorzugsweise ist M Lithium oder Natrium des Cyclopentadienyls mit der folgenden Formel G: 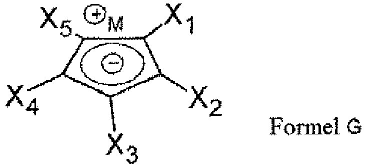 wobei X¹, X², X³, X⁴ und X⁵ wie in Anspruch 4 definiert sind,
c) Reaktion der Verbindung, erhalten in Schritt b), mit einem Eisensalz und der Verbindung D.

23. Verfahren zur Synthese der Verbindung mit der Formel (9), **dadurch gekennzeichnet, dass** es die Reaktionsschritte des Verfahrens nach Anspruch 22 umfasst, wobei in der Verbindung G, X¹ = X⁴ = H und X² = X³ = P(Ph)₂ und X⁵ = t-Bu und M = Li.

24. Verfahren zur Synthese der Verbindung mit der Formel (10), **dadurch gekennzeichnet, dass** es die Reaktionsschritte des Syntheseverfahrens nach Anspruch 22 umfasst, wobei in der Verbindung G, X¹ = X² = X⁴ = X⁵ = H und X³ = P(i-Pr)₂ und M = Li.

25. Verfahren zur Synthese einer Verbindung mit der Formel (11), **dadurch gekennzeichnet, dass** es die Reaktionsschritte des Syntheseverfahrens nach Anspruch 22 umfasst, wobei in der Verbindung G, X¹ = X² = X⁴ = X⁵ = H und X³ = P(R⁴)₂,wobei R⁴ ausgewählt ist aus einer Aryl- oder cyclischen oder acyclischen aliphatischen Gruppe mit C₃ bis C₇ oder einer linearen oder verzweigten Alkylgruppe mit C₁ bis C₃₀, vorzugsweise mit C₃ bis C₁₀, insbesondere mit C₃ bis C₇, gesättigt oder nicht gesättigt, und mindestens ein Heteroatom, vorzugsweise N und/oder O enthalten kann; vorzugsweise R⁴ eine Cyclohexyl-, Tertiobutyl- oder Phenyl- oder Furylgruppe ist, vorzugsweise durch mindestens eine Methyl-, Methoxygruppe, ein Halogenatom wie z. B. F, Cl, Br, I, vorzugsweise F oder Cl, oder eine CF₃-Gruppe substituiert.

26. Verfahren zur Synthese einer Verbindung mit der Formel (12), **dadurch gekennzeichnet, dass** es die Reaktionsschritte des Syntheseverfahrens nach Anspruch 22 umfasst, wobei in der Verbindung G, M = Li, X¹ = X² = X⁴ = H, X³ = P(R⁴)₂ und X⁵ = t-Bu, wobei R⁴ ausgewählt ist aus einer Aryl- oder cyclischen oder acyclischen aliphatischen Gruppe mit C₃ bis C₇ oder einer linearen oder verzweigten Alkylgruppe mit C₁ bis C₃₀, vorzugsweise mit C₃ bis C₁₀, insbesondere mit C₃ bis C₅, gesättigt oder nicht gesättigt, und mindestens ein Heteroatom, vorzugsweise N und/oder O enthalten kann; vorzugsweise R⁴ eine Isopropyl-, Cyclohexyl-, Tertiobutyl- oder Phenyl- oder Furylgruppe ist, optional substituiert, vorzugsweise durch mindestens eine Methyl-, Methoxygruppe oder ein Halogenatom wie z. B. F, Cl, Br, I, vorzugsweise F oder Cl, oder eine CF3-Gruppe.

27. Verfahren zur Synthese einer Verbindung mit der Formel (13), **dadurch gekennzeichnet, dass** es die Reaktionsschritte des Syntheseverfahrens nach Anspruch 22 umfasst, wobei in der Verbindung G, M = Li, X¹ = X² = X⁴ = H, X³ = P(R⁴)₂ und X⁵ = P(R⁶)₂, wobei R⁴ und R⁶ unabhängig voneinander ausgewählt sind aus einer Aryl- oder cyclischen oder acyclischen aliphatischen Gruppe mit C₃ bis C₇ oder einer linearen oder verzweigten Alkylgruppe mit C₁ bis C₃₀, vorzugsweise mit C₃ bis C₁₀, insbesondere mit C₃ bis C₅, gesättigt oder nicht gesättigt, und mindestens ein Heteroatom, vorzugsweise N und/oder O enthalten kann; vorzugsweise R⁴ und R⁵ unabhängig voneinander eine Isopropyl-, Cyclohexyl-, Tertiobutyl- oder Phenyl- oder Furylgruppe ist, optional substituiert, vorzugsweise durch mindestens eine Methyl-, Methoxygruppe oder ein Halogenatom wie z. B. F, Cl, Br, I, vorzugsweise F oder Cl, oder eine CF₃-Gruppe.

28. Verfahren zur Synthese einer Verbindung mit der Formel (14), **dadurch gekennzeichnet, dass** es die Reaktionsschritte des Syntheseverfahrens nach Anspruch 22 umfasst, wobei in der Verbindung G, M = Li, X¹ = X² = X⁴ = X⁵ = H, X² = C(CH₃)₂-P(R³)₂,und R³ ausgewählt ist aus einer Aryl- oder cyclischen oder acyclischen aliphatischen Gruppe mit C₃ bis C₇ oder einer linearen oder verzweigten Alkylgruppe mit C₁ bis C₃₀, vorzugsweise mit C₃ bis C₁₀, insbesondere mit C₃ bis C₅, gesättigt oder nicht gesättigt, und mindestens ein Heteroatom, vorzugsweise N und/oder O enthalten kann; vorzugsweise R³ eine Isopropyl-, Cyclohexyl-, Tertiobutyl- oder Phenyl- oder Furylgruppe ist, vorzugsweise durch mindestens eine Methyl-, Methoxygruppe, ein Halogenatom wie z. B. F, Cl, Br, I, vorzugsweise F oder Cl, oder eine CF₃-Gruppe substituiert.

29. Verfahren zur Synthese der Verbindung mit der Formel (15), **dadurch gekennzeichnet, dass** es die Reaktionsschritte des Syntheseverfahrens nach Anspruch 22 umfasst, wobei in der Verbindung G, M = Na, X¹ = X² = X⁴ = X⁵ = H, und X³ = CHO.

30. Verfahren zur Synthese einer Verbindung mit der Formel (16), **dadurch gekennzeichnet, dass** es die Reaktion der Verbindung mit der Formel (15) mit einem sekundären Amin in Anwesenheit von NaBH(OAc)₃ umfasst, wobei das sekundäre Amin die Formel HN(R⁵)₂ aufweist, wobei R⁵ eine Aryl- oder aliphatische Gruppe, zyklisch oder azyklisch, mit C₃ bis C₇ oder eine lineare oder verzweigte Alkylgruppe mit C₁ bis C₃₀, vorzugsweise mit C₃ bis C₁₀, insbesondere mit C₃ bis C₅, gesättigt oder nicht gesättigt, ist, und mindestens ein Heteroatom, vorzugsweise N und/oder O enthalten kann; vorzugsweise R⁵ eine Ethylgruppe ist.

31. Verfahren zur Synthese der Verbindungen mit den Formeln (17) und (18), **dadurch gekennzeichnet, dass** es die folgenden Schritte umfasst:
a) Synthese der Verbindung mit der folgenden Formel F: 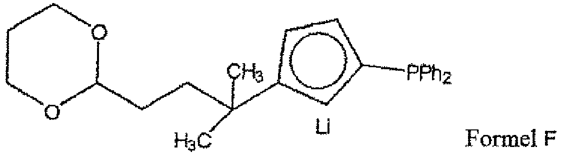
b) Synthese eines Salzes eines Metalls M, vorzugsweise ist M Lithium oder Natrium des Cyclopentadienyls mit der folgenden Formel G: 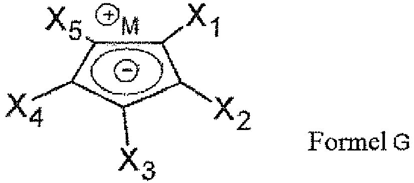 wobei X¹, X², X³, X⁴ und X⁵ wie oben in den Verbindungen mit der Formel (9) bis (14) für die Verbindungen mit den Formeln (17) oder (18) vom Typ unsymmetrische Tri- oder Diphosphine definiert sind,
c) Reaktion der Verbindung, erhalten in Schritt b), mit einem Eisensalz und der Verbindung F.

32. Verfahren zur Synthese von Metallkomplexen der Verbindungen mit den Formeln (1) bis (18), **dadurch gekennzeichnet, dass** es die Komplexierung jedes der Verbindungen mit den Formeln (1) bis (18) mit einem Metall umfasst, ausgewählt aus den Übergangsmetallen d, vorzugsweise Palladium, Kupfer, Nickel, Platin, Iridium, Rhodium, Eisen, Zirconium, Titan und Ruthenium, insbesondere Palladium.

33. Verfahren zur Synthese von Träger-Metallkomplexen der Verbindungen, die aus den Formeln (1) bis (18) stammen, **dadurch gekennzeichnet, dass** es das Implantieren auf einem Träger oder das Copolymerisieren jedes der Trägerkomplexe nach Anspruch 5 oder erhalten durch das Verfahren von Anspruch 32 umfasst.

34. Verwendung mindestens einer Verbindung nach einem beliebigen der Ansprüche 1 bis 4 zur Herstellung eines Trägerkatalysators.

35. Verwendung von mindestens einem Komplex nach Anspruch 5 zur Herstellung eines Trägerkatalysators.

36. Verfahren zur katalytischen Kopplung von nukleophilen und elektrophilen Verbindungen, vorzugsweise zur Kohlenstoff-Kohlenstoff-, Kohlenstoff-Stickstoff- und Kohlenstoff-Sauerstoff-Kopplung, **dadurch gekennzeichnet, dass** ein Trägerkatalysator oder Trägerkomplex nach Anspruch 6 oder erhalten durch das Verfahren nach Anspruch 33 bis 35 verwendet wird.

## Claims

1. Compounds of the following formula (I): 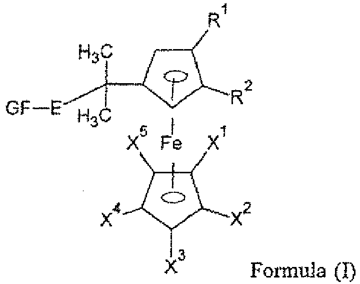 in which:
- E is a linear C₁ to C₅ alkyl chain,
- GF is a reactive group chosen from the vinyl and alkoxysilane groups the alkoxy chain of which is C₁ to C₅, linear or branched, saturated or unsaturated, optionally containing at least one heteroatom, preferably N and/or O;
- R¹ is a phosphine group P(R⁴)₂,
- R² is H or a phosphine group P(R⁶)₂,
- X¹, X², X³ and X⁴ are identical or different and are chosen, independently of one another, from a hydrogen atom, a group P(R⁸)₂, a group -C(CH₃)₂-P(R³)₂ or an amino group R⁷N(R⁵)₂*,*
- X₅ is either a group GF-E-C(CH₃)₂- in which GF and E are identical to GF and E defined previously, or H, or a phosphine group P(R¹⁰)₂ or an amino group R¹¹N(R⁹)₂,
- R³, R⁴, R⁵, R⁶, R⁷, R⁸, R⁹, R¹⁰ and R¹¹ are, independently of one another, chosen from a C₁ to C₃₀ alkyl group, preferably C₁ to C₁₀, more preferably C₁ to C₅, linear or branched, saturated or unsaturated, optionally containing at least one heteroatom, preferably an N and/or O atom, or an aryl group, or a cyclic or acyclic aliphatic group, C₃ to C₇, optionally containing a heteroatom, preferably an N and/or O atom and optionally substituted, preferably by at least one methyl, methoxy or halogen (F, Cl, Br, I) preferably F or Cl, or CF₃ group; they are preferably chosen from a -CH₂-, ethyl (Et), isopropyl (i-Pr), cyclohexyl (Cy) or tert-butyl (t-Bu) group, or a phenyl (Ph) or furyl (Fu) group, optionally substituted, preferably by at least one methyl, methoxy, halogen (F, Cl, Br, I) preferably F or CL, or CF₃ group; more preferably R³, R⁴, R⁶, R⁸ and R¹⁰, are a phenyl group, R⁵ and R⁹ are both an ethyl group, R⁷, R¹¹ are both a -CH₂- group.

2. Compounds according to claim 1, **characterised in that**
- in formula (I), X₅ is GF-E-C(CH₃)₂-,
- X¹ and X⁴ are H and **in that** they have the following formula (I-A): 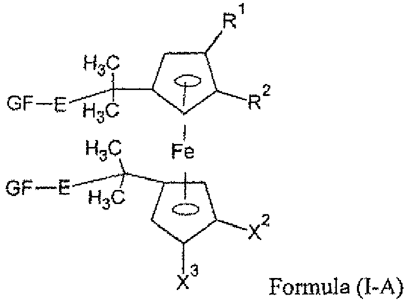 in which X² and X³ are as defined in claim 1.

3. Compounds according to claim 1 or claim 2, **characterised in that**, in formula (I) and in formula (I-A), X² = X³ = P(Ph)₂, X¹ and X⁴ are H and R¹ = R² = P(Ph)₂, and **in that** they have the following formulae (1) to (8), giving a symmetrical compound:

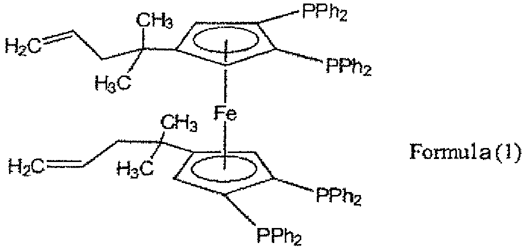

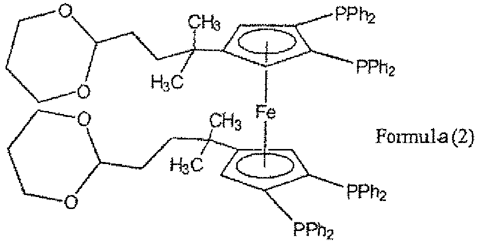

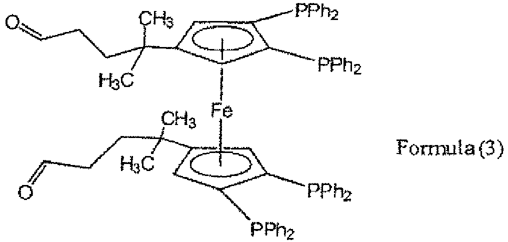

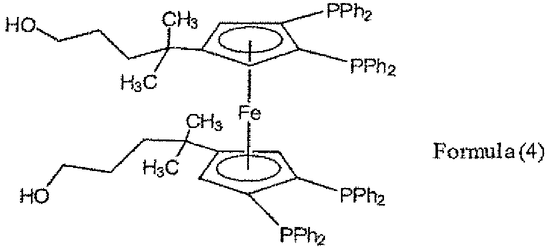

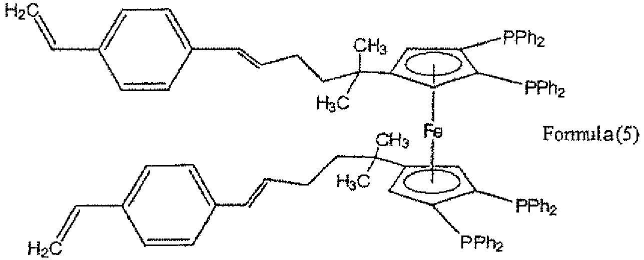

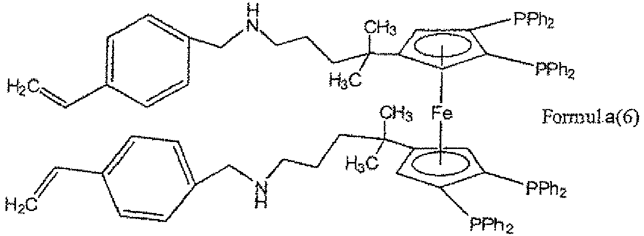

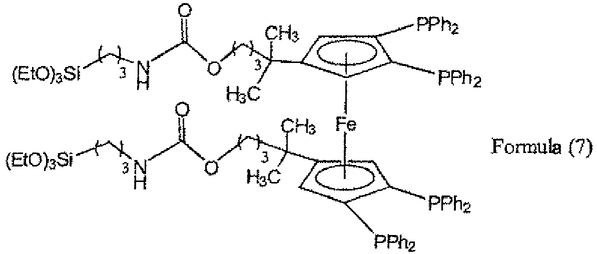

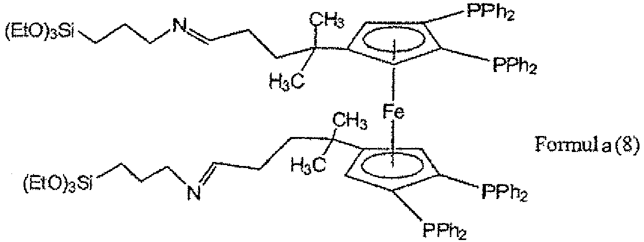

4. Compounds according to claim 1, **characterised in that**, in formula (I):
- X⁵ is different from GF-E-C(CH₃)₂, making them asymmetric, and **in that** they have the following formulae (9) to (18) in which:
- R⁴ and R⁶ designate, independently of one another, an isopropyl, cyclohexyl or tert-butyl group, or a phenyl or furyl group, optionally substituted, preferably by at least one methyl or methoxy group, a halogen atom such as F, Cl, Fb, I, preferably F or Cl, or a CF₃ group, and preferably R⁴ and R⁶ designate a phenyl group,
- R⁵ designates:
- either a C₁ to C₃₀ alkyl chain, preferably C₃ to C₁₀, more preferably C₃ to C₅, linear or branched, saturated or unsaturated, and which may contain at least one heteroatom, preferable N and/or O,
- or an aryl group, or a C₃ to C₇ cyclic or acyclic aliphatic group, optionally containing a heteroatom and optionally substituted, preferably by at least one methyl or methoxy group, a halogen atom such as F, Cl, Br, I, preferably F or Cl, or a CF₃ group, and preferably R⁵ designates an ethyl group, and **in that** they have the following formulae (9) to (18):

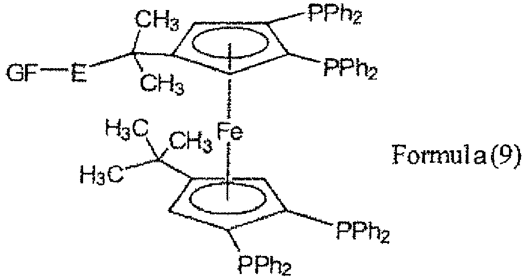

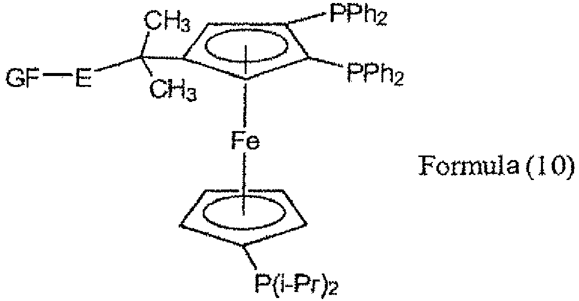

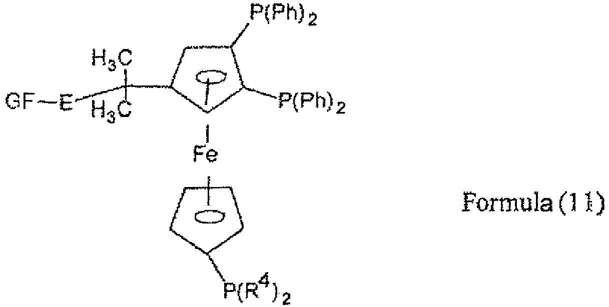

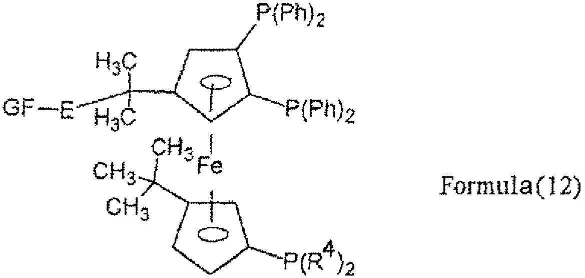

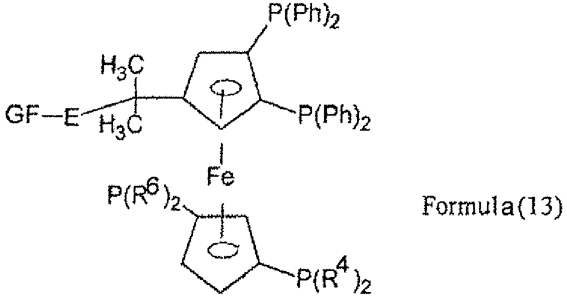

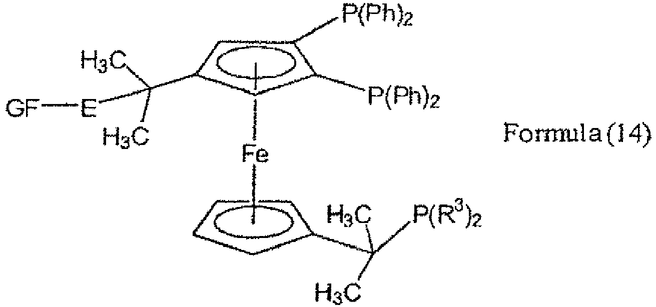

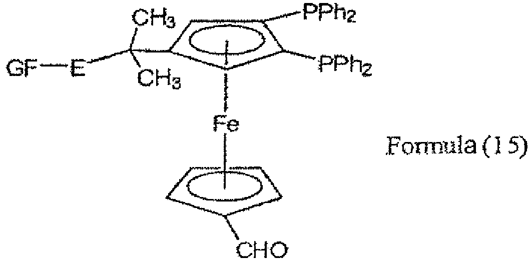

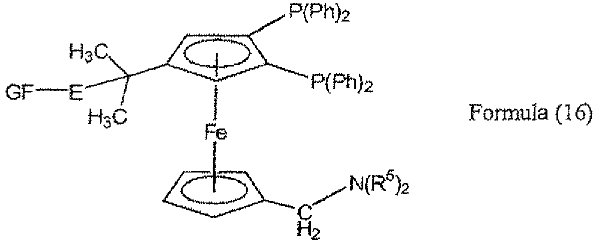

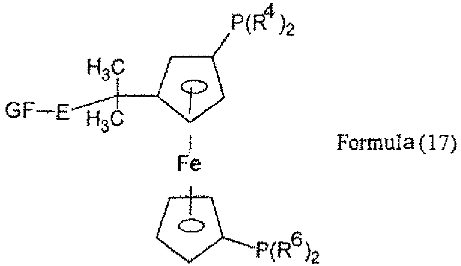

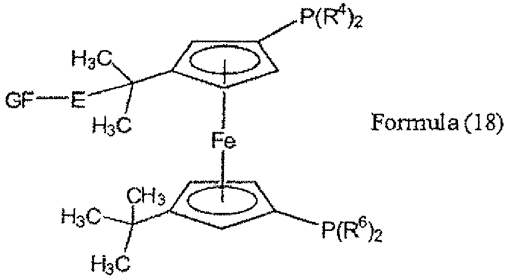

5. Complexes **characterised in that** they comprise a compound according to any of the preceding claims, complexed with a metal chosen from d-block transition metals, preferably from palladium, copper, nickel, platinum, rhodium, iridium, iron, zirconium, titanium and ruthenium and more preferably palladium.

6. Supported complexes **characterised in that** they comprise a complex according to claim 5 immobilised on a support by grafting or polymerisation of the reactive group GF of a compound according to any of claims 1 to 4 with a reactive group of a support.

7. Supported ligands **characterised in that** they comprise a compound according to any of claims 1 to 4 immobilised by grafting or polymerisation of its reactive group or groups GF with one of the reactive groups of a support.

8. Method for synthesising compounds according to claim 2 or claim 3, **characterised in that** it comprises the following steps:
a) preparation of a cyclopentadienyl lithium salt carrying the required groups E and GF or a reactive function that is a precursor of these groups,
b) two successive phosphination/lithiation reactions of the compound obtained at step a), and
c) formation of the required compound by reaction of the compound obtained at step b) with an iron salt.

9. Method for synthesising the compound of formula (1), **characterised in that** it comprises the following steps:
a) introduction of the functional group GF which is a vinyl group - CH=CH₂ and a spacer E which is -CH₂- by reaction of 6,6-dimethylfulvene with a methyl group the magnesium organic compound CH₂=CH-CH₂-MgCl in order to obtain 1-[2-(2-methyl)pent-4-enyl]cyclopentadiene,
b) lithiation of the product obtained at step a) with *n*-BuLi in order to obtain 1-[2-[2-methyl]pent-4-enyl]cyclopentadienyllithium,
c) two successive phosphinations, between which a lithiation step is performed with *n*-BuLi, of the product obtained at step b) with ClP(Ph)₂,
d) treatment of the compound obtained at step c) with *n*-BuLi to obtain 1,2-bis (diphenylphosphino)-4-[2-(2-methyl)pent-4-enyl] cyclopentadienyllithium, and
e) synthesis of the compound of formula (1) by reacting FeCl₂ with the compound obtained at step d).

10. Method for synthesising the compound of formula (2), **characterised in that** it comprises the following steps:
a) reaction of a magnesium organic compound of the following formula: 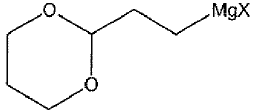 in which X is Br or UI
with CH₃COCl, to obtain 4-(1,3-dioxane-2-yl)butane-2-one of the following formula A: 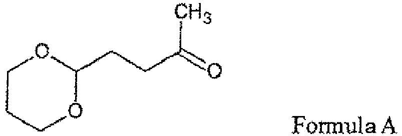
b) reaction of the compound of formula A obtained at step a), with pyrrolidine and cyclopentadienyllithium (CpLi) in order to obtain 2-[3-(cyclopenta-2,4-dienylidene)butyl]-1,3-dioxane of the following formula B: 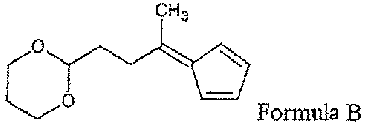
c) lithiation of the compound of formula B with methyllithium (MeLi) to obtain the compound of the following formula C: 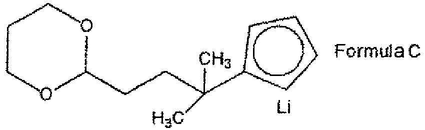
d) two successive phosphinations of the compound of formula C with ClP(Ph)₂, with intermediate treatment with lithium salt corresponding to monophosphination by the action of *n*-BuLi, followed by a treatment with *n*-BuLi to obtain the compound of the following formula D: 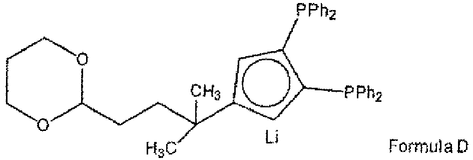
e) formation of the compound of the following (2): 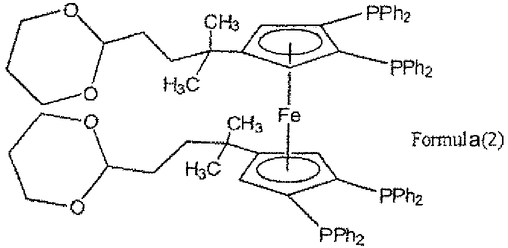 by reaction of the compound of formula D with an iron salt.

11. Compounds **characterised in that** they have one of the following formulae B or C or D or F:

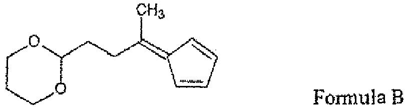

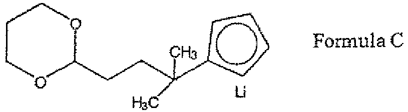

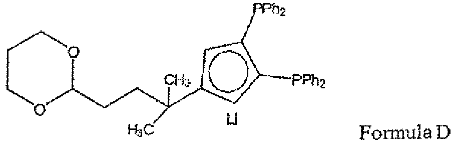

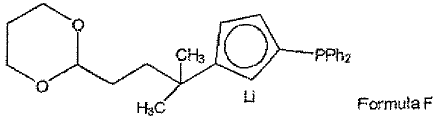

12. Method for synthesising the compound of formula (3), **characterised in that** it comprises the deprotection of the compound of formula (2) under acidic conditions.

13. Method for synthesising the supported compound of formula (3), **characterised in that** it comprises the deprotection of the compound of formula (2) under acidic conditions and the grafting of the compound of formula (3) thus obtained on a support comprising NH₂ groups.

14. Method for synthesising the compound of formula (4), **characterised in that** it comprises the reduction of the compound of formula (3).

15. Method for synthesising the supported compound of formula (4), **characterised in that** it comprises the grafting of the compound of formula (4) with a support comprising reactive groups able to react with an alcohol group, preferably isocyanate or silane groups.

16. Method for synthesising the compound of formula (5), **characterised in that** it comprises the reaction of the compound of formula (3) with a compound of the following formula (1-4):

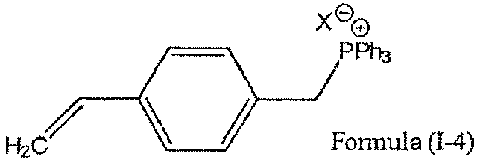

17. Method for synthesising the compound of formula (5), **characterised in that** it comprises the reaction of the compound of formula (3) with a compound of the following formula (I-5):

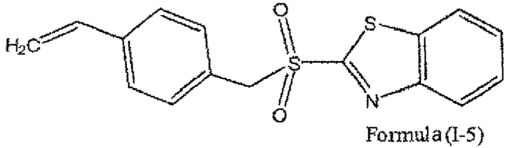

18. Method for synthesising the compound of formula (6), **characterised in that** it comprises the reaction of the compound of formula (3) with a p-vinylbenzylamine compound of the following formula (1-2):

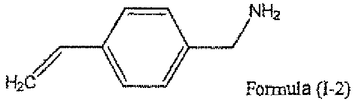

19. Method for synthesising the supported compound of formula (6), **characterised in that** it comprises the polymerisation of the compound of formula (6) with the styrene compound of the following formula (1-3):

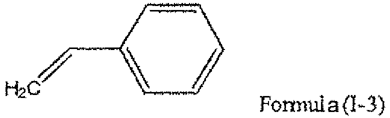

20. Method for synthesising the compound of formula (7), **characterised in that** it comprises the reaction of the compound of formula (4) with an alkoxysilane of the following formula (1-6):

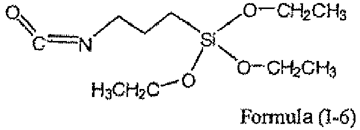

21. Method for synthesising the compound of formula (8), **characterised in that** it comprises the reaction of the compound of formula (3) with an alkoxysilane compounds of the following formula (I-7):

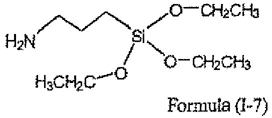

22. Method for synthesising a compound having one of formulae (9) to (16), **characterised in that** it comprises the following steps:
a) synthesis of the compound of the following formula D: 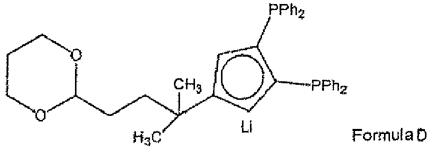
b) synthesis of a salt of a metal M, preferably M is lithium or sodium, of the cyclopentadienyl having the following formula G: 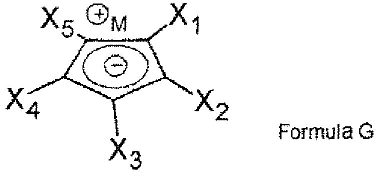 in which X¹, X², X³, X⁴ et X⁵ are as defined in claim 4,
c) reaction of the compound obtained at step b) with an iron salt and the compound D.

23. Method for synthesising the compound of formula (9), **characterised in that** it comprises the reaction steps of the method according to claim 22, in which, in the compound G, X¹ = X⁴ = H and X² = X³ = P(Ph)₂ and X⁵ = t-Bu and M = Li.

24. Method for synthesising the compound of formula (10), **characterised in that** it comprises the reaction steps of the synthesis method according to claim 22 in which, in the compound G, X¹ = X² = X⁴ = X⁵ = H and X³ = P(i-Pr)₂ and M = Li.

25. Method for synthesising a compound of formula (11), **characterised in that** it comprises the reaction steps of the synthesis method according to claim 22 in which, in the compound G, X¹ = X² = X⁴ = X⁵ = H and X³ = P(R⁴)₂, in which R⁴ is chosen from an aryl group or a C₃ to C₇ cyclic or acyclic aliphatic group, or a linear or branched C₁ to C₃₀ alkyl group, preferably C₃ to C₁₀, more preferably C₃ to C₇, saturated or unsaturated, and which may contain at least one heteroatom, preferably N and/or O; preferably R⁴ is a cyclohexyl or tert-butyl group, or a phenyl or furyl group, optionally substituted, preferably by at least one methyl or methoxy group, a halogen atom such as F, Cl, Br, I, preferably F or Cl, or a CF₃ group.

26. Method for synthesising a compound of formula (12), **characterised in that** it comprises the reaction steps of the synthesis method according to claim 22 in which, in the compound G, M = Li, X¹ = X² = X⁴ = H, X³ = P(R⁴)₂ and X⁵ = t-Bu, in which R⁴ is chosen from an aryl group or a C₃ to C₇ cyclic or acyclic aliphatic group, or a C₁ to C₃₀ linear or branched alkyl group, preferably C₃ to C₁₀, more preferably C₃ to C₅, saturated or unsaturated, and which may contain at least one heteroatom preferably N and/or O; preferably R⁴ is an isopropyl, cyclohexyl or tert-butyl group, or a phenyl or furyl group, optionally substituted, preferably by at least one methyl or methoxy group, or a halogen atom such as F, Cl, Fb, I, preferably F or Cl, or a CF₃ group.

27. Method for synthesising a compound of formula (13), **characterised in that** it comprises the reaction steps of the synthesis method according to claim 22, in which, in the compound G, M = Li, X¹ = X² = X⁴ = H, X³ = P(R⁴)₂ and X⁵ = P(R⁶)₂, in which R⁴ and R⁶ are, independently of each other, chosen from an aryl group or a C₃ to C₇ cyclic or acyclic aliphatic group, or a C₁ to C₃₀ linear or branched alkyl group, preferably C₃ to C₁₀, more preferably C₃ to C₅, saturated or unsaturated, and which may contain at least one heteroatom, preferably N and/or O; preferably R⁴ and R⁶ are, independently of each other, an isopropyl, cyclohexyl or tert-butyl group, or a phenyl or furyl group, optionally substituted, preferably by at least one methyl or methoxy group, or a halogen atom such as F, Cl, Br, I, preferable F or Cl, or a CF₃ group.

28. Method for synthesising a compound of formula (14), **characterised in that** it comprises the reaction steps of the synthesis method according to claim 22, in which, in the compound G, M = Li, X¹ = X² = X⁴ = X⁵ = H, X² = C(CH₃)₂-P(R³)₂ and R³ is chosen from an aryl group or a C₃ to C₇ cyclic or acyclic aliphatic group, or a C₁ to C₃₀ linear or branched alkyl group, preferably C₃ to C₁₀, more preferably C₃ to C₅, saturated or unsaturated, and which may contain at least one heteroatom, preferably N and/or O; preferably R³ is an isopropyl, cyclohexyl or tert-butyl group, or a phenyl or furyl group, optionally substituted, preferably by at least one methyl or methoxy group, or a halogen atom such as F, Cl, Br, I, preferably F or Cl, or a CF₃ group.

29. Method for synthesising the compound of formula (15), **characterised in that** it comprises the reaction steps of the synthesis method according to claim 22, in which, in the compound G, M = Na, X¹ = X² = X⁴ = X⁵ = H, and X³ = CHO.

30. Method for synthesising a compound of formula (16), **characterised in that** it comprises the reaction of the compound of formula (15) with a secondary amine in the presence of NaBH(OAc)₃, the secondary amine having the formula HN(R⁵)₂ in which R⁵ is an aryl group or a C₃ to C₇ cyclic or acyclic aliphatic group, or a C₁ to C₃₀ linear or branched alkyl group, preferably C₃ to C₁₀, more preferably C₃ to C₅, saturated or unsaturated, and which may contain at least one heteroatom, preferably N and/or O; preferably R⁵ is an ethyl group.

31. Method for synthesising compounds of formulae (17) and (18), **characterised in that** they comprise the following steps:
a) synthesis of the compound of the following formula F: 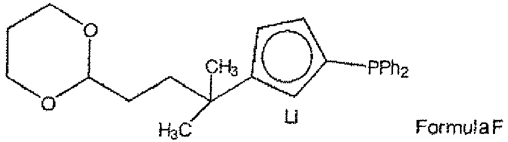
b) synthesis of a salt of a metal M, preferably lithium or sodium of cyclopentadienyl having the following formula G: 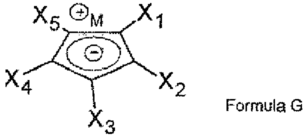 in which X¹, X², X³, X⁴ and X⁵ are as defined previously in the compounds of formula (9) to (14), for the compounds of formulae (17) or (18), of the asymmetric tri- or diphosphine type,
c) reaction of the compound obtained at step b) with an iron salt and the compound F.

32. Method for synthesising metal complexes of the compounds of formulae (1) to (18), **characterised in that** it comprises the complexing of each of the compounds of formulae (1) to (18) with a metal chosen from d-block transition metals, preferably palladium, copper, nickel, platinum, iridium, rhodium, iron, zirconium, titanium and ruthenium, more preferably palladium.

33. Method for synthesising supported metal complexes issuing from the compounds of formulae (1) to (18), **characterised in that** it comprises the grafting onto a support, or the copolymerisation, of each of the supported complexes according to claim 5 or obtained by the method of claim 32.

34. Use of at least one compound according to any of claims 1 to 4 for manufacturing a supported catalyst.

35. Use of at each one complex according to claim 5 for manufacturing a supported catalyst.

36. Method for the catalytic coupling of nucleophilic and electrophilic compounds, preferably carbon-carbon, carbon-nitrogen and carbon-oxygen coupling, **characterised in that** a supported catalyst or complex according to claim 6 or obtained by the method according to claims 33 to 35 is used.
